# EUROPEAN PATENT SPECIFICATION

(11) **EP 4 256 347 B1**
(45) Date of publication and mention of the grant of the patent: **16.07.2025**
(21) Application number: 21830394.9
(22) Date of filing: 01.12.2021
(51) Int. Cl.: G01N 33/68, G01N 33/74, C07C 317/04, C07C 317/14

(54) **DETECTION OF AN ANALYTE OF INTEREST BY CROSS SPRAY ESI MASS SPECTROMETRY**
NACHWEIS EINES ANALYTEN VON INTERESSE DURCH SPRÜHÜBERGREIFENDE ESI-MASSENSPEKTROMETRIE
DÉTECTION D'UN ANALYTE D'INTÉRÊT PAR SPECTROMÉTRIE DE MASSE ESI À PULVÉRISATION CROISÉE

(30) Priority: 03.12.2020 EP 20211558
(43) Date of publication of application: 11.10.2023
(73) Proprietor: F. Hoffmann-La Roche AG, 4070 Basel (CH); Roche Diagnostics GmbH, 68305 Mannheim (DE)
(72) Inventor: KARACA, Samir, 82377 Penzberg (DE); PIRKL, Nicole, 82377 Penzberg (DE); REMPT, Martin, 82377 Penzberg (DE)
(74) Representative: Epping, Claudia Viktoria
(86) International application number: PCT/EP2021/083681
(87) International publication number: WO 2022/117604

(56) References cited:
- WO-A1-2010/046482
- US-A1- 2002 011 560
- US-A1- 2016 284 529
- US-B2- 10 734 210
- T SANTA ET AL: "Derivatization reagents in liquid chromatography/electrospray ionization tandem mass spectrometry for biomedical analysis", DRUG DISCOVERIES & THERAPEUTICS, 1 October 2007 (2007-10-01), Japan, pages 108 - 118, XP055188450, Retrieved from the Internet <URL:http://www.ncbi.nlm.nih.gov/pubmed/22504396>
- CAROLIN EDINGER ET AL: "Electrochemical Deoxygenation of Aromatic Amides and Sulfoxides - Supporting information", EUROPEAN JOURNAL OF ORGANIC CHEMISTRY, vol. 2014, no. 24, 22 July 2014 (2014-07-22), DE, pages 1 - 33, XP055701013, ISSN: 1434-193X, DOI: 10.1002/ejoc.201402714
- MULLEN MATTHEW ET AL: "Combined secondary electrospray and corona discharge ionization (SECDI) for improved detection of explosive vapors using drift tube ion mobility spectrometry", TALANTA, ELSEVIER, AMSTERDAM, NL, vol. 209, 12 November 2019 (2019-11-12), XP085973405, ISSN: 0039-9140, [retrieved on 20191112], DOI: 10.1016/J.TALANTA.2019.120544
- KAMMEIJER GUINEVERE S. M. ET AL: "Dopant Enriched Nitrogen Gas Combined with Sheathless Capillary Electrophoresis-Electrospray Ionization-Mass Spectrometry for Improved Sensitivity and Repeatability in Glycopeptide Analysis", ANALYTICAL CHEMISTRY, vol. 88, no. 11, 7 June 2016 (2016-06-07), US, pages 5849 - 5856, XP055897723, ISSN: 0003-2700, Retrieved from the Internet <URL:https://pubs.acs.org/doi/pdf/10.1021/acs.analchem.6b00479> [retrieved on 20220304], DOI: 10.1021/acs.analchem.6b00479
- ALAGESAN KATHIRVEL ET AL: "To enrich or not to enrich: Enhancing (glyco)peptide ionization using the CaptiveSpray nanoBooster(TM)", BIORXIV, 5 April 2019 (2019-04-05), pages 1 - 20, XP055897721, Retrieved from the Internet <URL:https://www.biorxiv.org/content/10.1101/597922v1.full.pdf> [retrieved on 20220304], DOI: 10.1101/597922
- CRICK PETER J. ET AL: "Evaluation of novel derivatisation reagents for the analysis of oxysterols", BIOCHEMICAL AND BIOPHYSICAL RESEARCH COMMUNICATIONS, vol. 446, no. 3, 1 April 2014 (2014-04-01), Amsterdam NL, pages 756 - 761, XP055897726, ISSN: 0006-291X, DOI: 10.1016/j.bbrc.2014.01.173
- SANTA TOMOFUMI: "Derivatization reagents in liquid chromatography/electrospray ionization tandem mass spectrometry", BIOMEDICAL CHROMATOGRAPHY, vol. 25, no. 1-2, 5 November 2010 (2010-11-05), GB, pages 1 - 10, XP055897730, ISSN: 0269-3879, DOI: 10.1002/bmc.1548
- WANG HUIXIN ET AL: "Supercharging protein ions in native mass spectrometry using theta capillary nanoelectrospray ionization mass spectrometry and cyclic alkylcarbonates", ANALYTICA CHIMICA ACTA, ELSEVIER, AMSTERDAM, NL, vol. 1003, 12 December 2017 (2017-12-12), pages 1 - 9, XP085333444, ISSN: 0003-2670, DOI: 10.1016/J.ACA.2017.11.075
- EDINGER CAROLIN ET AL: "Electrochemical Deoxygenation of Aromatic Amides and Sulfoxides", EUROPEAN JOURNAL OF ORGANIC CHEMISTRY, vol. 2014, no. 24, 22 July 2014 (2014-07-22), DE, pages 5144 - 5148, XP055921485, ISSN: 1434-193X, DOI: 10.1002/ejoc.201402714

## Description

### Field of the Invention

The present invention relates to a method, a use of the method, and a use of a dopand for the enhancement of detection of an analyte of interest by Cross Spray ESI mass spectrometry.

### Background of the Invention

Mass spectrometry (MS) is a widely used technique for the qualitative and quantitative analysis of chemical substances ranging from small molecules to macromolecules. In general, it is a very sensitive and specific method, allowing even for the analysis of complex biological, for example (e.g.), environmental or clinical samples. However, for several analytes, especially if analysed from complex biological matrices such as serum, sensitivity of the measurement remains an issue.

Often MS is combined with chromatographic techniques, particularly gas and liquid chromatography such as e.g. HPLC. Here, the analysed molecule (analyte) of interest is separated chromatographically and is individually subjected to mass spectrometric analysis (Higashi et al. (2016) J. of Pharmaceutical and Biomedical Analysis 130 p. 181-190).

Kammeijer et al. (Anal Chem 88 2016, pp. 5849) describes dopant enriched nitrogen gas combined with sheathless CE-ESI-MS for improved sensitivity and repeatability in glycopeptide analysis.

Alagesan et al. (bioRxiv 2019 pp. 1) discloses an enhancing (glyco)peptide ionization using the CaptiveSpray nanoBooster^{™}.

US 2016/0284529 A1 discloses high resolution mobility analysis of large charge-reduced electrospray ions.

US 10 734 210 B2 discloses mass spectrometer and operating methods thereof.

US 2002/0011560 A1 discloses apparatus and method for focusing ions and charged particles at atmospheric pressure.

Crick Peter J. et al. (vol. 446, no. 3, 1 April 2014) discloses the evaluation of novel derivatisation reagents for the analysis of oxysterols.

Santa Tomofumi (vol. 25, no. 1-2, 5 November 2010 (2010-11-05), pages 1-10) discloses derivatization reagents in liquid chromatography/electrospray ionization tandem mass spectrometry.

Wang et al. (Analytica Chimica Acta, Vol. 1003, 12 December 2017 (2017-12-12), pages 1-9) discloses supercharging protein ions in native mass spectrometry using theta capillary nanoelectrospray ionization mass spectrometry and cyclic alkylcarbonates.

Edinger et al. (European Journal of organic Chemistry, Vol. 2014, no. 24, 22 July 2014 (2014-07-22), pages 5144-5148) discloses electrochemical deoxygenation of aromatic amides and sulfoxides.

WO 2010/046482 A1 discloses high purity diphenyl sulfone, preparation and use thereof for the preparation of a poly(aryletherketone).

To ensure reliable and sensitive mass spectrometric detection (avoiding matrix effects and interference as well as increasing sensitivity) it is necessary to separate chromatographically the target analytes as well as possible. In general, this can be done by isocratic or gradient systems, for example, reversed phase HPLC columns and gradients from aqueous to organic phases. The columns used for HPLC require flow rates between 0.1 and 1.0 ml/min. Under these optimal flow conditions, very narrow chromatographic peaks with very small peak volumes are produces.

ESI (electrospray ionization) is a technique used in mass spectrometry to produce ions using an electrospray in which a high voltage is applied to a liquid to create an aerosol or a gas.

There is, however, still a need of increasing the sensitivity of MS analysis methods, particularly for the analysis of interest that have a low abundance or when only little materials (such as biopsy tissues) are available.

To add a DMSO to an LC-MS eluent are known to enhance the sensitivity for peptide signals. To add dopands in the liquid phase like DMSO can change chromatographic parameters like retention time and are disadvantage, if different solvents needs to go onto this HPLC system because of cleaning all tubings from the previous solvent with the dopand e.g. DMSO. Further, if the dopand (e.g. DMSO) is within the eluent the dopand get into the ion source permanently and will therefore contaminate over time the ion source. The mixture behavior of the dopand and the respective eluent need to match in a way that precipitation or reaction effects are vanished. If a post-column infusion of any dopand is used a chromatographic peak width extension is caused. This is disadvantage if very sharp peak shapes are resulted from (modern) UHPLC-separations and or ultra-short gradient times.

If reactants for derivatization are infused before the ion source, the risk of contamination of the analyte ESI emitter is high as well as peak width problems occure.

There is thus an urgent need in the art for a method which allows for a sensitive detection of analytes from complex biological matrices as well as exhibiting a chemical structure which does not negatively influence the MS measurement workflow. This is of particular importance in a random-access, high-throughput MS set up, wherein several different analytes exhibiting different chemical properties have to be measured in a short amount of time.

The present invention relates to a method of determining the level of an analyte of interest in a sample which allows for a sensitive determination of analyte molecules such as steroids, proteins, and other types of analytes, in biological samples. The reagent is designed in a modular manner to allow the individual adaption for specific needs arising in the measurement of certain analytes or for specifc workflow adaptations.

It is an object of the present invention to provide a method, a use of the method, and a use of a dopand for the enhancement of detection of an analyte of interest by Cross Spray ESI mass spectrometry.

This object is or these objects are solved by the subject matter of the independent claims. Further embodiments are subjected to the dependent claims.

### Summary of the Invention

In the following, the present invention relates to the following aspects:
In a first aspect, the present invention relates to a method for determining the presence or the level of an analyte of interest in a sample comprising the following steps:
a) Providing a first electrospray ionization source for a first analytical flow stream comprising the analyte of interest,
b) Providing a second electrospray ionization source for a second analytical flow stream comprising a dopand or a derivatization reagent, wherein each of the first analytical flow stream and the second analytical flow stream is comprised in gaseous form or aerosol,
c) Intermixing the first and the second analytical flow stream for forming a mixture or a derivatized analyte of interest, wherein the mixture comprises the analyte of interest and the dopand, and
d) Determining the presence or the level of an analyte of interest in the sample using mass spectrometry.

In a second aspect, the present invention relates to the use of the method of the first aspect of the present invention for determining the presence or the level of an analyte of interest in a sample.

In a third aspect, the present invention relates to a use of a dopand in a method according to the first aspect of the present invention for enhancing the sensitivity of mass spectrometric detection of an analyte of interest comprises formula I:

R1-SO₂-R2 (I)

wherein R1, R2 are each independently selected from alkyl or aryl, wherein alkyl comprises one to six C-atoms, wherein aryl comprises one to six C-atoms.

### List of Figures

**Figure** 1 shows a schematic view of a diagnostic system according to the present invention.
**Figure 2** shows the TIC (Total Ion Count) in % vs. retension time of mixtures comripsing petides as analytes of interests and DMSO as a dopand in different concentrations (0% (without DMSO), 10%, 20% and 30% (v/v)).
**Figure 3** shows the TIC in % vs. retension time of mixtures comprising toramycin as analytes of interests and DMSO as a dopand in different concentrations (0% (without DMSO), 5% and 30% (v/v)) .
**Figures 4A to 4D** show the TIC in % vs. retension time of mixtures comprising estradiol as an analyte of interests with or without ammoniumfluoride as a dopand.
**Figure 5** shows the TIC in % vs. retension time of testosterone with an hydrazon derivatization reagent for coupling.
**Figure 6** shows the TIC in % vs. retension time of testosterone with a derivatization reagent.
**Figure 7** shows the fold change of the peptide mixtures (purchased by Sigma Aldrich) respective peptides against a cross flow eluent of the blank H₂O/CAN (CAN = acetonitrile).
**Figure 8** shows a schematic experiment set-up: The peptid test mixture is mixed with the dopand after the column and before the mass spectrometer in a reaction coil.
**Figure 9** shows the fold change of three peptides as analytes of interest by the addition of different dopands like DMSO, sulfolane, diphenylsulfone, 1,4 dioxane and propyleneglycole sulfite.

### Detailed Description of the Invention

Before the present invention is described in detail below, it is to be understood that this invention is not limited to the particular embodiments and examples described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The various described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

### Definitions

The word "**comprise**", and variations such as "comprises" and "comprising", will be understood to imply the inclusion of a stated integer or step or group of integers or steps but not the exclusion of any other integer or step or group of integers or steps. The term "including" and "comprising" can be used interchangeable.

As used in this specification and the appended claims, the singular forms "**a**", "**an**", and "**the**" include plural referents, unless the content clearly dictates otherwise.

**Percentages, concentrations, amounts, and other numerical data** may be expressed or presented herein in a "range" format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "4% to 20 %" should be interpreted to include not only the explicitly recited values of 4 % to 20 %, but to also include individual values and sub-ranges within the indicated range. Thus, included in this numerical range are individual values such as 4, 5, 6, 7, 8, 9, 10, ... 18, 19, 20 % and sub-ranges such as from 4-10 %, 5-15 %, 10-20%, etc. This same principle applies to ranges reciting minimal or maximal values. Furthermore, such an interpretation should apply regardless of the breadth of the range or the characteristics being described.

The term "**about**" when used in connection with a numerical value is meant to encompass numerical values within a range having a lower limit that is 5% smaller than the indicated numerical value and having an upper limit that is 5% larger than the indicated numerical value.

In the context of the present invention, the term "**compound**" or "**derivatisation reagent**" or "**label**" or "**derivatisation agent**" are used interchangeably and refer to a chemical substance having a specific chemical structure. Said compound may comprise one or more reactive groups. Each reactive group may fulfil a different functionality, or two or more reactive groups may fulfil the same funtion. Reactive groups include but are not limited to reactive units, charged units, and neutral loss units. The derivatization reagent can undergo an atmospheric pressure chemical reaction with the analyte of interest.

The term "**Mass Spectrometry**" ("Mass Spec" or "MS") or "mass spectrometric determination" or "mass spectrometric analysis" relates to an analytical technology used to identify compounds by their mass. MS is a methods of filtering, detecting, and measuring ions based on their mass-to-charge ratio, or "m/z". MS technology generally includes (1) ionizing the compounds to form charged compounds; and (2) detecting the molecular weight of the charged compounds and calculating a mass-to-charge ratio. The compounds may be ionized and detected by any suitable means. A "mass spectrometer" generally includes an ionizer and an ion detector. In general, one or more molecules of interest are ionized, and the ions are subsequently introduced into a mass spectrographic instrument where, due to a combination of magnetic and electric fields, the ions follow a path in space that is dependent upon mass ("m") and charge ("z"). The term "ionization" or "ionizing" refers to the process **o**f generating an analyte ion having a net charge equal to one or more units. Negative ions are those having a net negative charge of one or more units, while positive ions are those having a net positive charge of one or more units. The MS method may be performed either in "negative ion mode", wherein negative ions are generated and detected, or in "positive ion mode" wherein positive ions are generated and detected.

"**Tandem mass spectrometry**" or "MS/MS" involves multiple steps of mass spectrometry selection, wherein fragmentation of the analyte occurrs in between the stages. In a tandem mass spectrometer, ions are formed in the ion source and separated by mass-to-charge ratio in the first stage of mass spectrometry (MS1). Ions of a particular mass-to-charge ratio (precursor ions or parent ion) are selected and fragment ions (or daughter ions) are created by collision-induced dissociation, ion-molecule reaction, or photodissociation. The resulting ions are then separated and detected in a second stage of mass spectrometry (MS2).

Since a mass spectrometer separates and detects ions of slightly different masses, it easily distinguishes different isotopes of a given element. Mass spectrometry is thus, an important method for the accurate mass determination and characterization of analytes, including but not limited to low-molecular weight analytes, peptides, polypeptides or proteins. Its applications include the identification of proteins and their post-translational modifications, the elucidation of protein complexes, their subunits and functional interactions, as well as the global measurement of proteins in proteomics. De novo sequencing of peptides or proteins by mass spectrometry can typically be performed without prior knowledge of the amino acid sequence.

Most sample workflows in MS further include sample preparation and/or enrichment steps, wherein e.g. the analyte(s) of interest are separated from the matrix using e.g. gas or liquid chromatography. Typically, for the mass spectrometric measurement, the following three steps are performed:
1. a sample comprising an analyte of interest is ionized, usually by complex formation with cations, often by protonation to cations. Ionization source include but are not limited to electrospray ionization (ESI), nano electrospray ionization (nanoESI) and atmospheric pressure chemical ionization (APCI).
2. the ions are sorted and separated according to their mass and charge. High-field asymmetric-waveform ion-mobility spectrometry (FAIMS) may be used as ion filter.
3. the separated ions are then detected, e.g. in multiple reaction mode (MRM), and the results are displayed on a chart.

The term "**electrospray ionization**" or "ESI," refers to methods in which a solution is passed along a short length of capillary tube, to the end of which is applied a high positive or negative electric potential. Solution reaching the end of the tube is vaporized (nebulized) into a jet or spray of very small droplets of solution in solvent vapor. This mist of droplets flows through an evaporation chamber, which is heated slightly to prevent condensation and to evaporate solvent. As the droplets get smaller the electrical surface charge density increases until such time that the natural repulsion between like charges causes ions as well as neutral molecules to be released.

The term "**nano electrospray ionization**" or "**nanoESI**" refers to methods typically using flow rates below 1 µL/min either in static or dynamic mode. Preferably, nanoESI uses a flow rate of 50 to 500 nl/min, e.g. 500 nl/min. 500 nl/min is equal to 0.5 µl/min.

The term "**atmospheric pressure chemical ionization**" or "APCI," refers to mass spectrometry methods that are similar to ESI; however, APCI produces ions by ion-molecule reactions that occur within a plasma at atmospheric pressure. The plasma is maintained by an electric discharge between the spray capillary and a counter electrode. Then ions are typically extracted into the mass analyzer by use of a set of differentially pumped skimmer stages. A counterflow of dry and preheated Ni gas may be used to improve removal of solvent. The gas-phase ionization in APCI can be more effective than ESI for analyzing less-polar entity.

"**High-field asymmetric-waveform ion-mobility spectrometry (FAIMS)**" is an atmospheric pressure ion mobility technique that separates gas-phase ions by their behavior in strong and weak electric fields.

"**Multiple reaction mode**" or "MRM" is a detection mode for a MS instrument in which a precursor ion and one or more fragment ions arc selectively detected.

Mass spectrometric determination may be combined with additional analytical methods including chromatographic methods such as gas chromatography (GC), liquid chromatography (LC), particularly HPLC, and/or ion mobility-based separation techniques.

In the context of the present disclosure, the term "**analyte**", "analyte molecule" , or "**analyte(s) of interest**" or "**analyte(s) of interests**" are used interchangeably referring the chemical specis to be analysed via mass spectrometry, in particular nanoESI mass spectrometry. Chemical specis suitable to be analysed via mass spectrometry, i.e. analytes, can be any kind of molecule present in a living organism, include but are not limited to nucleic acid (e.g. DNA, mRNA, miRNA, rRNA etc.), amino acids, peptides, proteins (e.g. cell surface receptor, cytosolic protein etc.), metabolite or hormones (e.g. testosterone, estrogen, estradiol, etc.), fatty acids, lipids, carbohydrates, steroids, ketosteroids, secosteroids (e.g. Vitamin D), molecules characteristic of a certain modification of another molecule (e.g. sugar moieties or phosphoryl residues on proteins, methyl-residues on genomic DNA) or a substance that has been internalized by the organism (e.g. therapeutic drugs, drugs of abuse, toxin, etc.) or a metabolite of such a substance. Such analyte may serve as a biomarker. In the context of present invention, the term "biomarker" refers to a substance within a biological system that is used as an indicator of a biological state of said system.

The term "**dopand**" can mean in this context a chemical substance that interacts with the respective analyte by undergoing atmospheric pressure chemical interactions with the analyte of interest.

The term "**permanent charge**" or "permanent charged" is used in the context of the present disclosure that the charge, e.g. a positive or negative charge, of a unit is not readily reversible, for example, via flushing, dilution, filtration, and the like. A permanent charge may be the result, for example, of covalently bonding. A reversible charge (a non-permanent charge) may be the result in contrast to a permanent charge, for example, of an electrostatic interaction.

The term "**permanent net charge**" or "net charged" is used in the context of the present disclosure that the permanent net charge is the total permanent charge an ion or molecule has. Permanent net charge can be calculated as follows: number of protons - number of electrons = permanent net charge. A permanent net charge can be seen as a covalent combination of atoms which forms by bond rearrangements a charged mojety in the molecule (e.g. quarternary nitrogen, tetramethylammonium) while a net charge can also exsist by addition or the abstraction of atoms e.g. hydrogen to result in a pseudomolecular ion consisting of [M⁺H]⁺ or [M⁻H]⁻. For Example, if the compound hast wo permanent positive charges and one permanent negative charge, then the permanent net charge is +1 (2*(+1)+(-1)=(+1)).

The term "**compound is capable of covalently binding to the analyte**" means that the compound is suitable to bind to the analyte. The binding between the compound and the analyte is covalent.

The term "**mass**", for example, m1, m2, m3, m4 or mx with x >4, represents the atomic mass, in particular the unified atomic mass. The unit of the unified atomic mass is u. In the biomedical field Dalton [Da] instead of the unified atomic mass [u] can be used. The Dalton is not an SI unit. The dalton is equivalent to unified atomic mass in that there is no conversion factor between these units. A "**mass spectrum**" is the two-dimensional representation of signal intensity (ordinate) versus m/z (abscissa). The position of a peak, as signals are usually called, reflects the m/z of an ion that has been created from the compound, analyte or combinations thereof (complex) within the ion source. The intensity of this peak correlates to the abundance of that ion. Often but not necessarily, the peak at highest m/z results from the detection of the intact ionized molecule, the molecular ion, M⁺. The molecular ion peak is usually accompanied by several peaks at lower or higher m/z caused by fragmentation of the compound, analyt or complex to yield fragment ions. Consequently, the respective peaks in the mass spectrum may be referred to as fragment ion peaks or daughter ion peaks. m/z is dimensionless by definition.

The term "**fragmentation**" can mean that the compound, analyt and/or complex is dissociated and form ions, e.g. at least one daughter ion, by passing the compound, analyt and/or complex in the ionization chamber of a mass spectrometer. The fragments cause a unique pattern in the mass spectrum. The term "fragmentation" can refer to the dissociation of a single molecule into two or more separate molecules. As used herein, the term fragmentation refers to a specific fragmentation event, wherein the breaking point in the parent molecule at which the fragmentation event takes place is well defined, and wherein the two or more daughter molecules resulting from the fragmentation event are well characterised. It is well-known to the skilled person how to determine the breaking point of a parent molecule as well as the two or more resulting daughter molecules. The resulting daughter molecules may be stable or may dissociate in subsequent fragmentation events. Exemplified, in case a parent molecule undergoing fragmentation comprises a N-benzylpyridinium unit, the skilled person is able to determine based on the overall structure of the molecule whether the pyridinium unit will fragment to release an benzyl entity or would be released completely from the parent molecule, i.e the resulting daughter molecules would either be an benzyl molecule and a parent molecule lacking of benzyl. Fragmentation may occur via collision-induced dissociation (CID), electron-capture dissociation (ECD), electron-transfer dissociation (ETD), negative electron-transfer dissociation (NETD), electron-detachment dissociation (EDD), photodissociation, particularly infrared multiphoton dissociation (IRMPD) and blackbody infrared radiative dissociation (BIRD), surface-induced dissociation (SID), Higher-energy C-trap dissociation (HCD), charge remote fragmentation.

The term "**m1/z1** < **m2/z2**" means that the mass-to-charge ratio of the compound (m1/z1) is smaller than the mass-to-charge ratio of at least one or exact one daughter ion of the compound (m2/z2).

The term "**limit of detection**" or "LOD" is the lowest concentration of an analyte that the bioanalytical procedure can reliably differentiate the analyte from background noise.

The term "**signal-to-noise ratio**" or S/N describes the uncertainty of an intensity measurement and provides a quantitative measure of a signal's quality by quantifying the ratio of the intensity of a signal relative to noise.

Analytes may be present in a sample of interest, e.g. a biological or clinical sample. The term "**sample**" or "sample of interest" are used interchangeably herein, referring to a part or piece of a tissue, organ or individual, typically being smaller than such tissue, organ or individual, intended to represent the whole of the tissue, organ or individual. Upon analysis a sample provides information about the tissue status or the health or diseased status of an organ or individual. Examples of samples include but are not limited to fluid samples such as blood, serum, plasma, synovial fluid, spinal fluid, urine, saliva, and lymphatic fluid, or solid samples such as dried blood spots and tissue extracts. Further examples of samples are cell cultures or tissue cultures.

A "**covalent bond**" or "**covalently linked**" or "**covalently bonded**" is at least one chemical bond that involves the sharing of electron pairs between atoms or molecules, e.g. between the compound and the analyte.

The terms "**compound**" and "**label**" or "derivatization reagent" can be used interchangeable.

Numerical values, e.g. 1, 2, 3, 4, 5 or 6, for the charges, e.g. z1, z2, z3, z4 or zx with x > 4, are absolute values of the charges. For example, net charge z1 = 2 can mean that the net charge z1 is +2 or the net charge is -2. Preferably, the charges in this case are positive numerical values, e.g. 2 = +2.

In this context "**level**" or "**level value**" encompasses the absolute amount, the relative amount or concentration as well as any value or parameter which correlates thereto or can be derived therefrom.

The term "**determining**" the level of the analyte of interest, as used herein refers to the quantification of the analyte of interest, e.g. to determining or measuring the level of the analyte of interest in the pretreated sample. The level of the analyte of interest is determined by nanoESI mass spectrometry.

In this context "**pretreated sample**" refers to a sample, which is prepared for the mass spectrometry, in particular the nanoESI mass spectrometry. In particular, pretreated sample is a sample, which is provided and/or prepared before step (a) and/or (b) of the method is performed. Before the analyte is being analysed via Mass Spectrometry, a sample may be pre-treated in a sample- and/or analyte specific manner. In the context of the present disclosure, the term "pre-treatment" or "pre-treated" refers to any measures required to allow for the subsequent analysis of a desired analyte via Mass Spectrometry, in particular NanoESI Mass Spectrometry. Pre-treatment measures typically include but are not limited to the elution of solid samples (e.g. elution of dried blood spots), addition of hemolizing reagent (HR) to whole blood samples, and the addition of enzymatic reagents to urine samples. Also the addition of internal standards (ISTD) is considered as pre-treatment of the sample. In particular, pre-treatment of the sample does not include enrichment step, e.g. by using magnetic or paramagnetic beads.

The term "hemolysis reagent" (HR) refers to reagents which lyse cells present in a sample, in the context of this invention hemolysis reagents in particular refer to reagents which lyse the cell present in a blood sample including but not limited to the erythrocytes present in whole blood samples. A well known hemolysis reagent is water (H₂O). Further examples of hemolysis reagents include but are not limited to deionized water, liquids with high osmolarity (e.g. 8M urea), ionic liquids, and different detergents.

Typically, an "internal standard" (ISTD) is a known amount of a substance which exhibits similar properties as the analyte of interest when subjected to the mass spectrometric detection worklflow (i.e. including any pre-treatment, enrichment and actual detection step). Although the ISTD exhibits similar properties as the analyte of interest, it is still clearly distinguishable from the analyte of interest. Exemplified, during a chromatographic separation, such as gas or liquid chromatography, the ISTD has about the same retention time as the analyte of interest from the sample. Thus, both the analyte and the ISTD enter the mass spectrometer at the same time. The ISTD however, exhibits a different molecular mass than the analyte of interest from the sample. This allows a mass spectrometric distinction between ions from the ISTD and ions from the analyte by means of their different mass/charge (m/z) ratios. Both are subject to fragmentation and provide daughter ions. These daughter ions can be distinguished by means of their m/z ratios from each other and from the respective parent ions. Consequently, a separate determination and quantification of the signals from the ISTD and the analyte can be performed. Since the ISTD has been added in known amounts, the signal intensity of the analyte from the sample can be attributed to a specific quantitative amount of the analyte. Thus, the addition of an ISTD allows for a relative comparison of the amount of analyte detected, and enables unambiguous identification and quantification of the analyte(s) of interest present in the sample when the analyte(s) reach the mass spectrometer. Typically, but not necessarily, the ISTD is an isotopically labeled variant (comprising e.g. ²H, ¹³C, or ¹⁵N etc. label) of the analyte of interest.

In addition to the pre-treatment, the sample may also be subjected to one or more enrichment steps. In the context of the present disclosure, the term "first enrichment process" or "**first enrichment workflow**" refers to an enrichment process which occurs subsequent to the pre-treatment of the sample and provides a sample comprising an enriched analyte relative to the initial sample. The first enrichment workflow may comprise chemical precipitation (e.g. using acetonitrile) or the use of a solid phase. Suitable solid phases include but are not limited to Solid Phase Extraction (SPE) cartridges, and beads. Beads may be non-magnetic, magnetic, or paramagnetic. Beads may be coated differently to be specific for the analyte of interest. The coating may differ depending on the use intended, i.e. on the intended capture molecule. It is well-known to the skilled person which coating is suitable for which analyte. The beads may be made of various different materials. The beads may have various sizes and comprise a surface with or without pores.

In the context of the present disclosure the term "**second enrichment process**" or "second enrichment workflow" refers to an enrichment process which occurs subsequent to the pre-treatment and the first enrichment process of the sample and provides a sample comprising an enriched analyte relative to the initial sample and the sample after the first enrichment process.

In the context of the present disclosure, the sample may be derived from an "**individual**" or "subject". Typically, the subject is a mammal. Mammals include, but are not limited to, domesticated animals (e.g., cows, sheep, cats, dogs, and horses), primates (e.g., humans and non-human primates such as monkeys), rabbits, and rodents (e.g., mice and rats).

The term "**serum**" as used herein is the clear liquid part of the blood hat can be separated from clotted blood. The term "**plasma**" as used herein is the clear liquid part of blood which contains the blood cells. Serum differs from plasma, the liquid portion of normal unclotted blood containing the red and white cells and platelets. It is the clot that makes the difference between serum and plasma. The term "**whole blood**" as used herein contains all components of blood, for examples white and red blood cells, platelets, and plasma.

The term "**in vitro method**" is used to indicate that the method is performed outside a living organism and preferably on body fluids, isolated tissues, organs or cells.

The term "**automatically**" or "**automated**" as used herein is a broad term and is to be given its ordinary and customary meaning to a person of ordinary skill in the art and is not to be limited to a special or customized meaning. The term specifically may refer, without limitation, to a process which is performed completely by means of at least one computer and/or computer network and/or machine, in particular without manual action and/or interaction with a user.

The term "**chromatography**" refers to a process in which a chemical mixture carried by a liquid or gas is separated into components as a result of differential distribution of the chemical entities as they flow around or over a stationary liquid or solid phase.

The term "**liquid chromatography**" or "LC" refers to a process of selective retardation of one or more components of a fluid solution as the fluid uniformly percolates through a column of a finely divided substance, or through capillary passageways. The retardation results from the distribution of the components of the mixture between one or more stationary phases and the bulk fluid, (i.e., mobile phase), as this fluid moves relative to the stationary phase(s). Methods in which the stationary phase is more polar than the mobile phase (e.g., toluene as the mobile phase, silica as the stationary phase) are termed normal phase liquid chromatography (NPLC) and methods in which the stationary phase is less polar than the mobile phase (e.g., water-methanol mixture as the mobile phase and C18 (octadecylsilyl) as the stationary phase) is termed reversed phase liquid chromatography (RPLC).

"**High performance liquid chromatography**" or "HPLC" refers to a method of liquid chromatography in which the degree of separation is increased by forcing the mobile phase under pressure through a stationary phase, typically a densely packed column. Typically, the column is packed with a stationary phase composed of irregularly or spherically shaped particles, a porous monolithic layer, or a porous membrane. HPLC is historically divided into two different sub-classes based on the polarity of the mobile and stationary phases.

Further well-known LC modi include hydrophilic interaction chromatography (HILIC), size-exclusion LC, ion exchange LC, and affinity LC.

LC separation may be single-channel LC or multi-channel LC comprising a plurality of LC channels arranged in parallel. In LC analytes may be separated according to their polarity or log P value, size or affinity, as generally known to the skilled person.

The term "**reactive unit**" refers to a unit able to react with another molecule, i.e. which is able to form covalent bond with another molecule, such as an analyte of interest. Typically, such covalent bond is formed with a chemical group present in the other molecule. Accordingly, upon chemical reaction, the reactive unit of the compound forms a covalent bond with a suitable chemical group present in the analyte molecule. As this chemical group present in the analyte molecule, fulfils the function of reacting with the reactive unit of the compound, the chemical group present in the analyte molecule is also referred to as the "functional group" of the analyte. The formation of the covalent bond occurs in each case in a chemical reaction, wherein the new covalent bond is formed between atoms of the reactive group and the functional groups of the analyte. It is well known to the person skilled in the art that in forming the covalent bond between the reactive group and the functional groups of the analyte, atoms are lost during this chemical reaction.

The term "**analytical flow stream is comprised in gaseous form or aerosol**" can mean in this context that aerosol consist of mico droplets, e.g. >1nm while in gaseous state or gaseous form the analyte or analytes are free or only surrounded by solvate shell and forming micro compartments < 1nm. The named size means the particle diameter.

In the context of the present disclosure, the term "**complex**" or "derivatized analyte of interest" are used interchangeable and refers to the product produced by the reaction of a compound with an analyte molecule. This reaction leads to the formation of a covalent bond between the compound and the analyte. Accordingly, the term complex refers to the covalently bound reaction product formed by the reaction of the compound with the analyte molecule.

In the context of the present invention, the method of the first aspect of the present invention can also be called as a method with a cross flow spray configuration.

### Embodiments

The method for determining the presence or the level of an analyte of interest in a sample comprises the following steps:
a) Providing a first electrospray ionization source for a first analytical flow stream comprising the analyte of interest,
b) Providing a second electrospray ionization source for a second analytical flow stream comprising a dopand or a derivatization reagent, wherein each of the first analytical flow stream and the second analytical flow stream is comprised in gaseous form or aerosol,
c) Intermixing the first and the second analytical flow stream for forming a mixture or a derivatized analyte of interest, wherein the mixture comprises the analyte of interest and the dopand, and
d) Determining the presence or the level of an analyte of interest in the sample using mass spectrometry.

The amount or concentration or level of the analyte, in particular the relative amount of the analyte in the pretreated sample may be determined. The method is highly accurate and gives coefficient of variation (CV) of 20% or less, particularly of 10% or less, more particularly 2% or less, e.g. 1% to 2% when repeatedly determining the amount of the analyte.

The sample may be a pretreated sample.

The method may be an in vitro method.

The sample may be obtained from a patient sample, which is selected from a group consisting of serum, plasma and whole blood sample from an individual.

The sample may be a human sample, preferably a hemolysed whole-blood sample, particularly a hemolysed human whole-blood sample.

The sample may be a hemolysed whole-blood sample, particularly a hemolysed human whole-blood sample, e.g. derived from a subject the blood of which to be tested for the amount of the analyte of interest. Hemolysis is particularly carried out by dilution with water (H₂O), e.g. deionized or distilled water, in particular in a ratio of sample: water of about 1:2 to about 1:20, in particular about 1:5 to about 1: 10, in particular about 1:9 (v/v). The sample may be hemolysed for a time less than about 30 min, less than about 10 min, less than about 5 min or even less than about 2 min. In particular embodiments, the sample is hemolyzed for a time of about 10 to about 60 sec.

The hemolysis may be carried out by mixing sample and water, in particular by vortexing sample and water. In particular sample and water are mixed, in particular vortexed, for about 1 to about 60 sec, in particular for about 5 to about 30 sec, in particular for about 10 sec.

During hemolysis the sample may be kept at a temperature of 20 °C to 30 °C, in particular at 22 °C to 25 °C, in particular at room temperature.

The hemolysis of the sample may be carried out by mixing the sample with water in a ratio of 1:9 by vortexing for 10 sec at room temperature.

According to step (a), a first electrospray ionization source is provided. The first electrospray ionization source is for a first analytical flow stream. The first analytical flow stream comprises the analyte of interest.

The first electrospray ionization source may be designed in such a way that ions are generated by a ESI suppored tip by forming a Taylor cone which is capable to reach for the generated ions the entrance of the mass spectrometer. The ion source can be closed as well as connected to surrounding air. Material can be stainless steele, glass or non polarizable material in general not to disturb electicla fields.

The first analytical flow stream is comprised in gaseous form or aerosol.

According to step (b), a second electrospray ionization source is provided. The second electrospray ionization source is for a second analytical flow stream. The second analytical flow stream comprises a dopand or a derivatization reagent.

The second analytical flow stream is comprised in gaseous form or aerosol.

According to step (c), the first and the second analytical flow stream for forming a mixture or a derivatized analyte of interest are intermixed. The mixture comprises the analyte of interest and the dopand. The intermixing step comprises the the first and second analytical flow stream are crossed, preferably for signal enhancement.

A mixture is formed in step (c). The mixture comprises or consists of the at least one analyte of interest and the dopand.

A derivatized analyte of interest or at least derivatized analyte of interest is formed in step (c). The derivatized analyte of interest may result from reacting the at least one analyte of interest with the derivatization reagent.

An internal standard may be provided. The internal standard (preferably an isotopically labelled analyte) may be dissolved in an appropriate solvent and added to the sample in a defined concentration.

The method may comprise a further step c1) after step c),
c1) Introducing the mixture or a derivatized analyte of interest in a unit for performing mass spectrometry.

The method may be performed for enhancing the sensitivity of the mass spectrometric detection, preferably for enhancing the signal by enhancing the numbers of analytes which are ionized.

The method may be performed for enhancing the mass spectrometric signal of the analyte of interest.

In step b) a derivatization reagent for chemical reaction with the analyte of interest may be added.

In step b) a dopand for ion enhancement may be added.

The first analytical flow stream may be chromatographically purified, preferably before the first analytical flow stream passes the first electrospray ionization source.

The first analytical flow stream may be intermixed with the second analytical flow stream after leaving the first and the second electrospray ionization source.

The first analytical flow stream and/or the second analytical flow stream may have a flow rate in the range of 1 to 1000 µL/min, preferably in the range of 50 to 500 µL/min.

The first electrospray ionization source and/or the second electrospray ionization source may be a borosilicate glass capillary and/or a metal based capillary.

The first electrospray ionization source and/or the second electrospray ionization source may be an ESI or nanoESI.

The first electrospray ionization source and the second electrospray ionization source may be arranged at least 10° to 180° according a plan view over the two electrospray ionization sources and/or at least 10° to 180° according to a front view for the electrospray ionization sources. Preferably, the first electrospray ionization source and the second electrospray ionization source are arranged at least 70° to 100°, e.g. 90° according a plan view over the two electrospray ionization sources and/or at least 70° to 100°, e.g. 90° according to a front view for the electrospray ionization sources.

The first and the second analytical flow stream may be mixed, preferably without blocking by a rotable baffle.

The dopand may be DMSO or NH₄F.

The second flow stream may comprise a concentration of the dopand or a concentration of the derivatization reagent in the range of 5 to 35 % (v/v).

The dopand may be DMSO having a concentration in the range of 5 to 35 % (v/v).

Before step (a), the first analytical flow stream comprising the analyte of interest may undergo a chromatographic step that comprises at least one or more methods selected from the following group: chromatography, high performance liquid chromatography (HPLC), liquid chromatography high performance liquid chromatography (LC-HPLC), gel permeation chromatography (GPC), flash chromatography. Chromatography is, for example, size exclusion chromatography.

The method may be automated.

According to step (b), the analyte of interest in the sample may be derivatised.

Step (b) may be performed by a derivatization reagent.

Step (b) may be performed in a time range of 5 minutes at the maximum, preferably 3 minutes at the maximum, more preferably 2 minutes at the maximum.

The compound may be cabable of covalently binding to the analyte or is covalently bounded to the analyte.

The analyte of interest may be derivatized in step (b) by a derivatization reagent, which is capable of forming a covalent binding to the analyte of interest, in particular wherein after step (b) the derivatization reagent may be covalently bound to the analyte of interest for forming a derivatized analyte of interest. A complex of the analyte and compound may be formed.

The derivatization reagent may be single permanent positive charged or single permanent negative charged.

The derivatization reagent may be double permanent positive charged or double permanent negative charged.

The derivatization reagent may be more than twice permanent positive charged, e.g. 3, 4, 5, 6 or 7, or more than twice permanent negative charged, e.g. 3, 4, 5, 6 or 7.

The derivatization reagent may be free of a permanent charge.

The derivatization reagent may have a net charge z1, in particular before fragmentation. After fragmentation the compound can be splitted or cleaved into at least one daughter ion. The daughter ion may have a net charge z2, which is smaller than the net charge z1 (z2 < z1). A complex comprising or constisting of the analyte and the compound may have a net charge z3, in particular before fragmentation. After fragmentation, the complex can be splitted or cleaved into at least one daughter ion having a net charge z4, which is smaller than the net charge z3 (z4 < z3). At least one daughter ion can mean in this context that one daughter ion or more are formed after fragmentation. The one daughter ion and the other daughter ions may differentiate from each other at least by their mass, charge or structure.

The derivatization reagent may comprise a permanent charge, in particular a permanent net charge, wherein said compound may be capable of covalently binding to the analyte of interest,
wherein said derivatization reagent may have a mass m1 and a net charge z1,
wherein the derivatization reagent may be capable of forming at least one daughter ion having a mass m2 < m1 and a net charge z2 < z1 after fragmentation by mass spectrometric determination,
wherein m1/z1 < m2/z2.

The compound may be selected from the the following group:

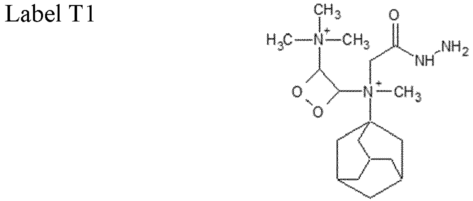

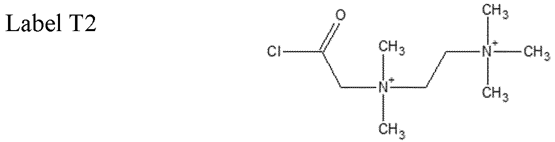

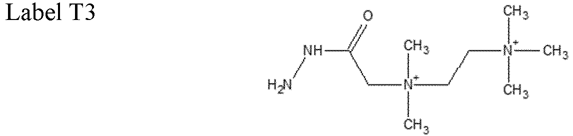

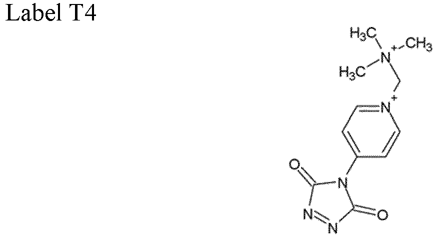

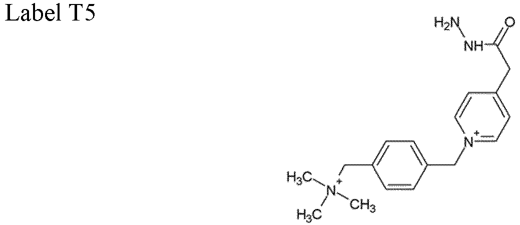

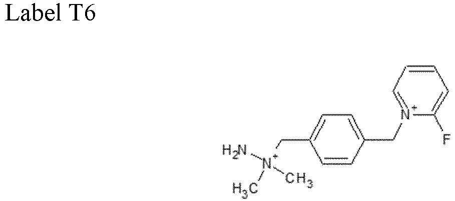

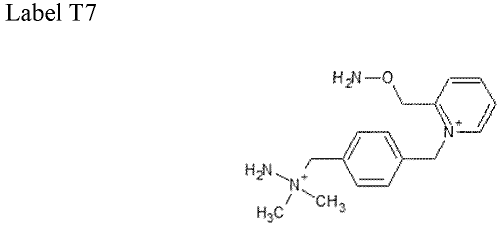

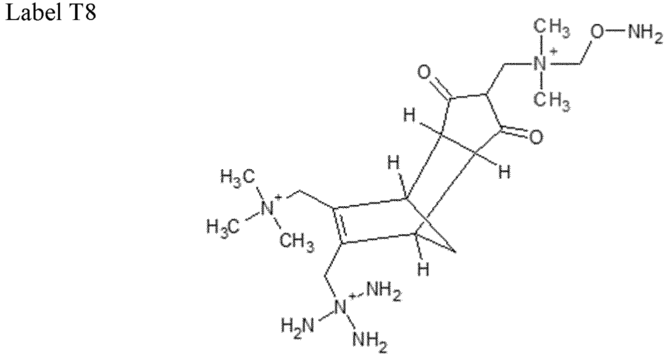

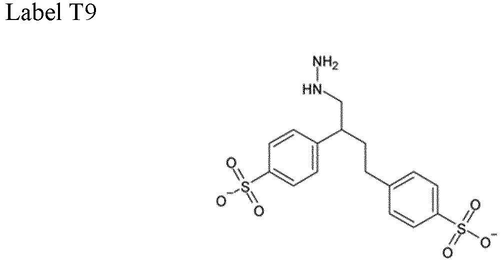

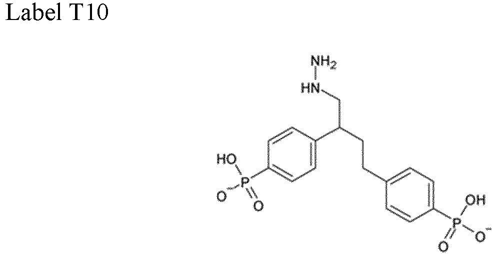

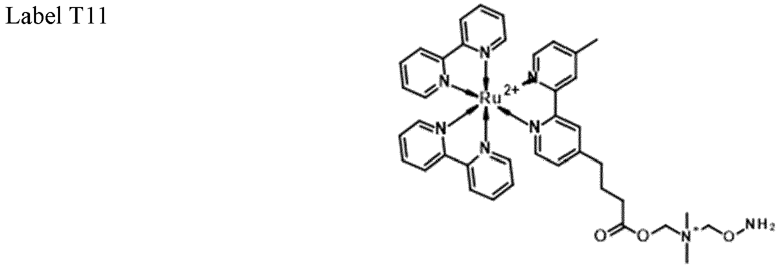

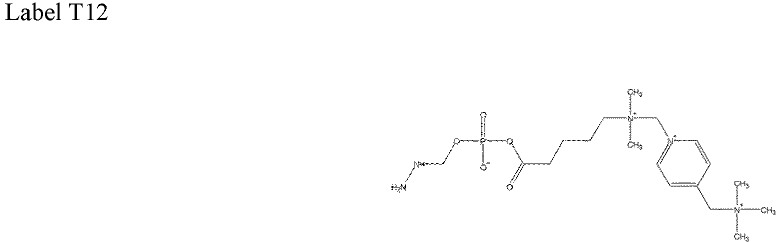

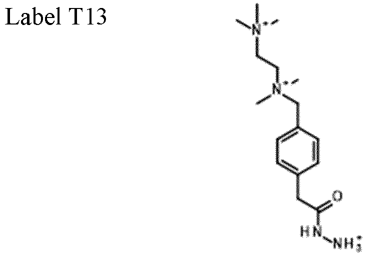

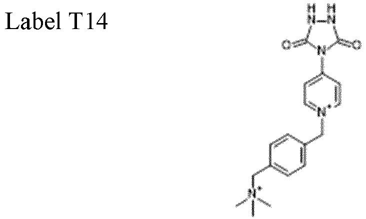

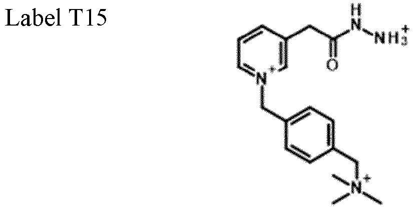

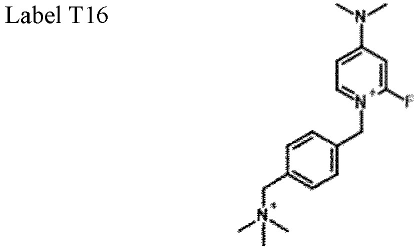

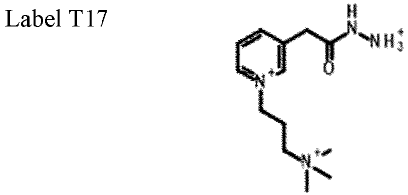

The derivatization reagent may comprise a reactive unit K, which is able of reacting with a carbonyl group, phenol group, amine, hydroxyl group or diene group of the analyte of interest.

K may be selected from the group consisting of hydrazide, hydrazine, hydroxylamine, Br, F-aromatic, 4-substituted 1,2,4-triazolin-3,5-dione (TAD), active ester, sulfonylchloride and reactive carbonyl.

The derivatization reagent may comprise a counter ion for forming a salt, wherein the counter ion may be preferably selected from the following group: Cl⁻, Br⁻, F⁻, formiate, trifluoroacetate, PF₆⁻, sulfonate, phosphate, acetate.

Step b) may be performed at a temperature, which is at least 20 °C or more.

Step b) may be performed at least at 30 °C, for example 35 °C.

Step b) may be performed at least at 40 °C, for example 45 °C.

Step b) may be performed at least at 50 °C, for example 55 °C.

Step b) may be performed at least at 60 °C, for example 65 °C.

Step b) may be performed at least at 70 °C, for example 75 °C.

Step b) may be performed at least at 80 °C, for example 85 °C.

Step b) may comprises the addition of a further substance or further substances. This further substance or these further substances may be, e.g. additives. The further substance or the further substances may be, for example, for protonation and/or for catalysis. In particular the further substance or the further substances for catalysis may be (a) lewis base(s).

A further substance or further substances for protonation may be selected from the group consisting of protonating organic acids, e.g. formic acid.

A further substance or further substances for catalysis may be selected from the group consisting of lewis bases, e.g. phenylenediamine.

The derivatization reagent may comprise formula A or B: 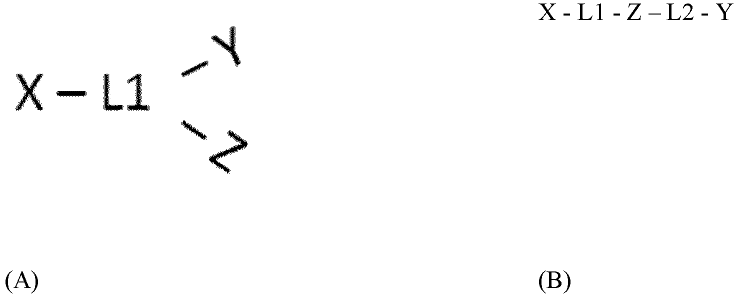 wherein
X may be a reactive unit, which may in particular be capable of forming a covalent bond with an analyte of interest,
L1 and L2 may be independently of each other substituted or unsubstituted linker, in particular branched or linear linker,
Y may be a neutral loss unit, and
Z may be a charged unit comprising at least one permanently charged moiety, in particular comprising one permanently charged moiety,
including any salt thereof.

The derivatization reagent of formula A may be selected from the group consisting of 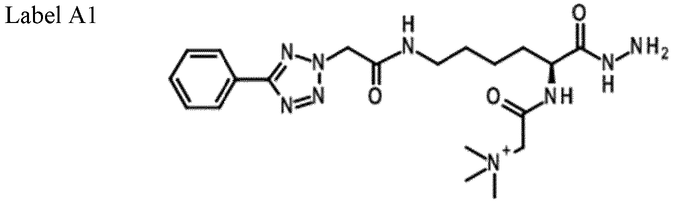 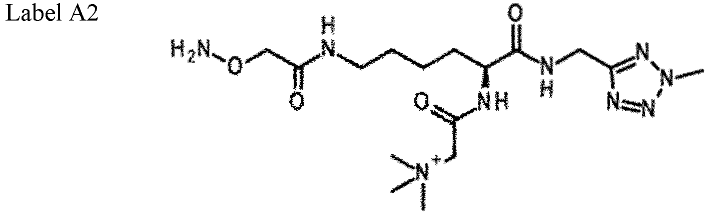 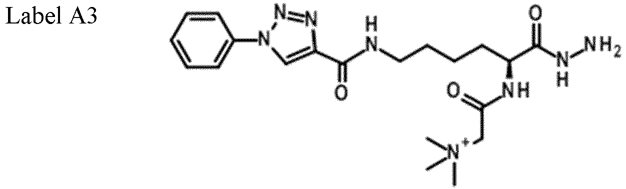 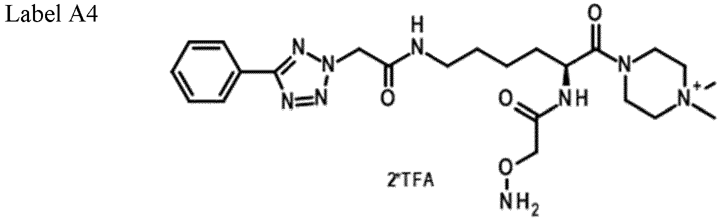 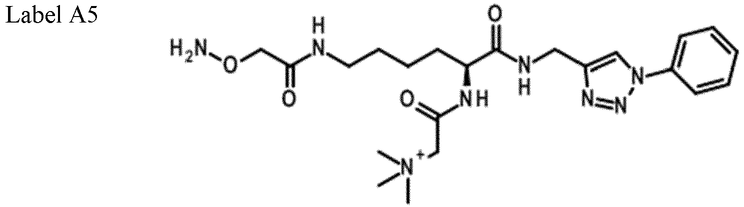 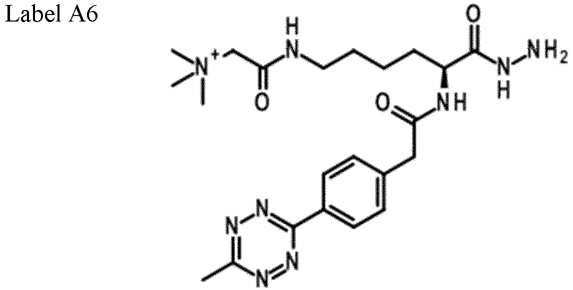 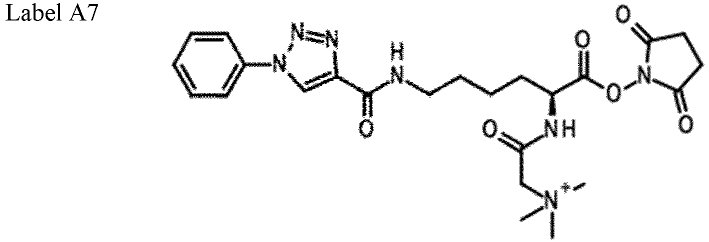 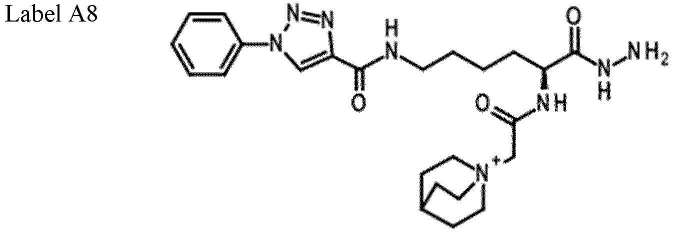 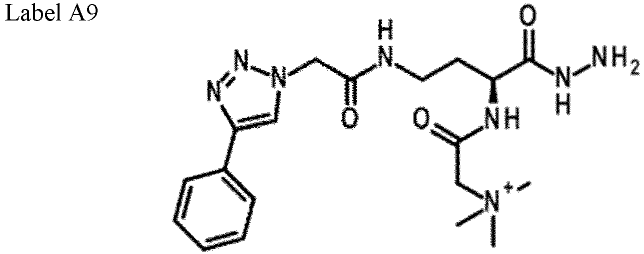 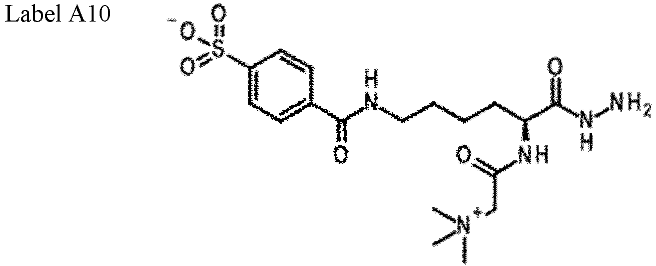 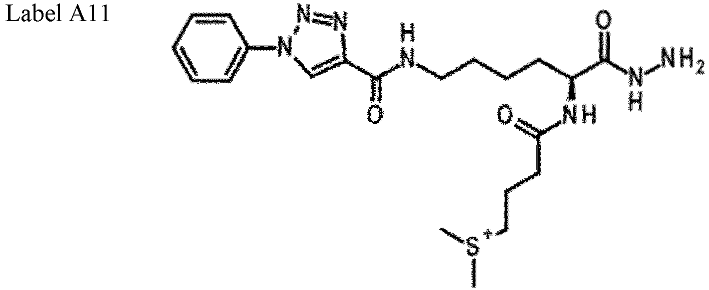 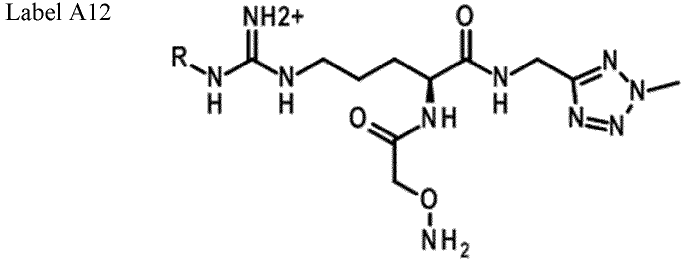 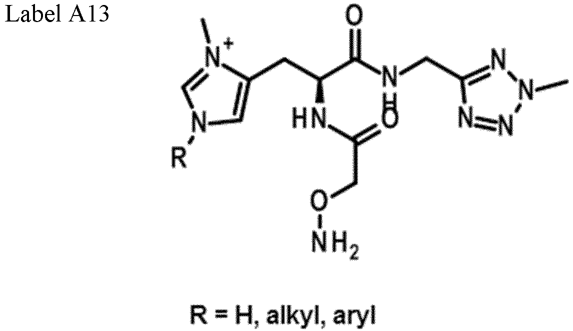 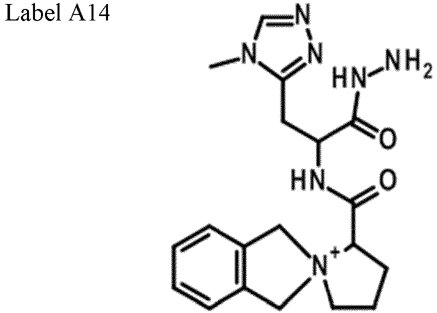 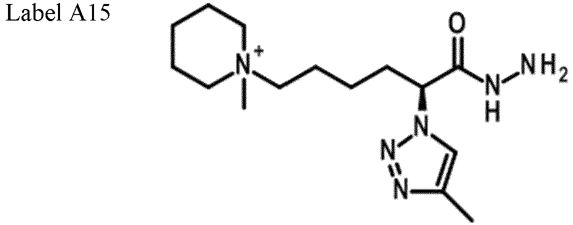 or combinations thereof.

The derivatization reagent of formula B may be selected from the group consisting of 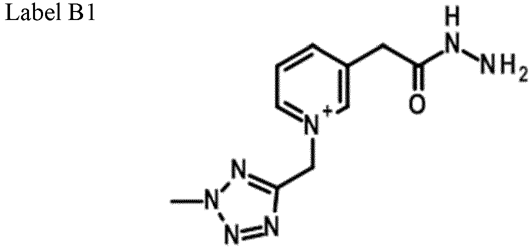 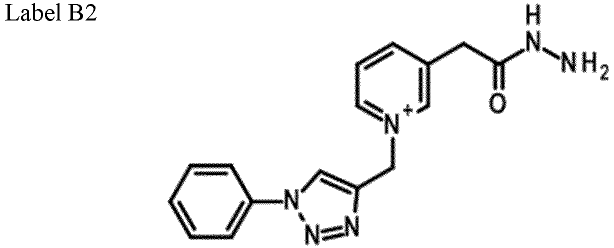 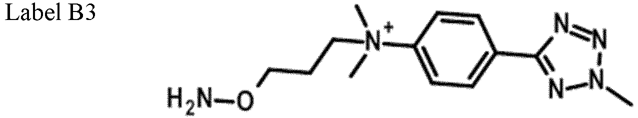 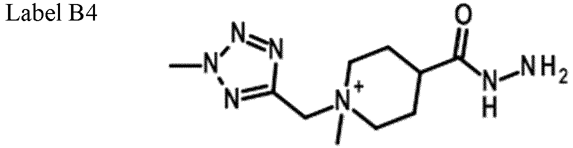 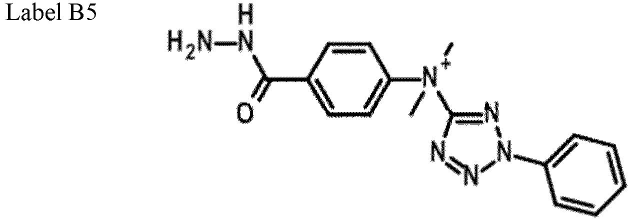 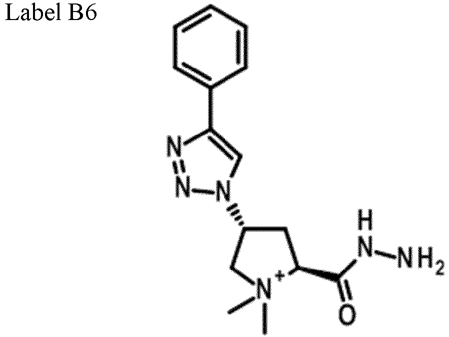 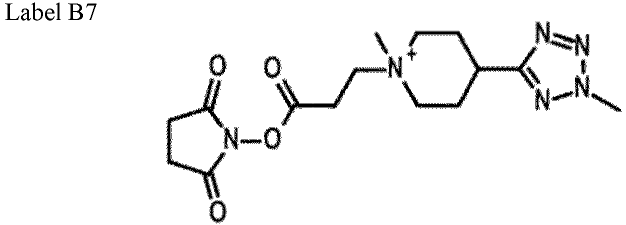 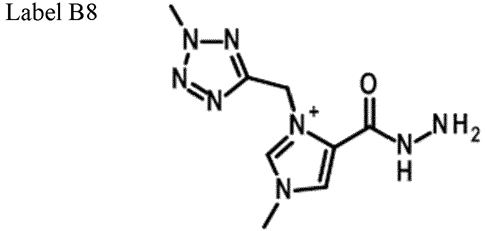 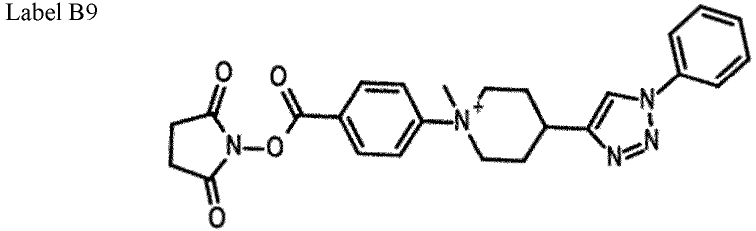 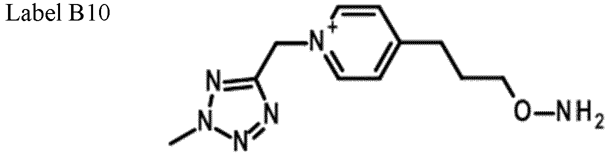 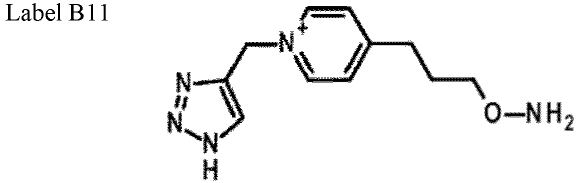 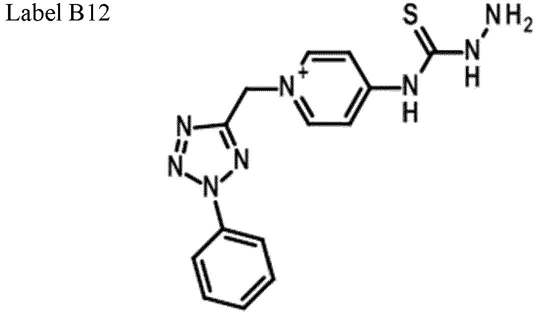 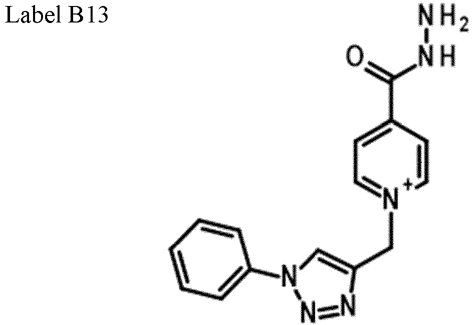 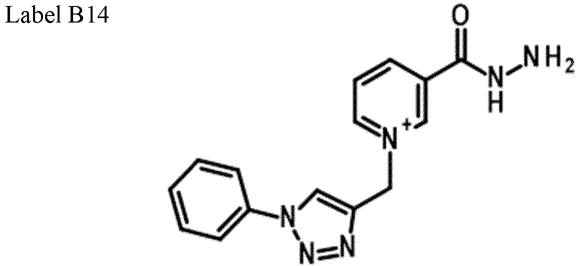 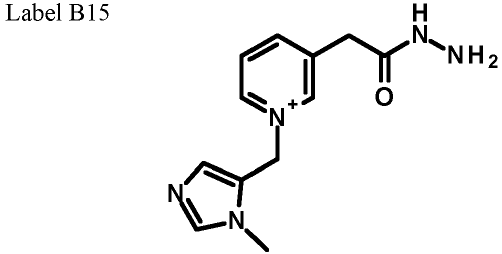 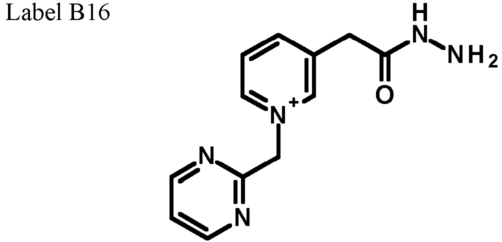 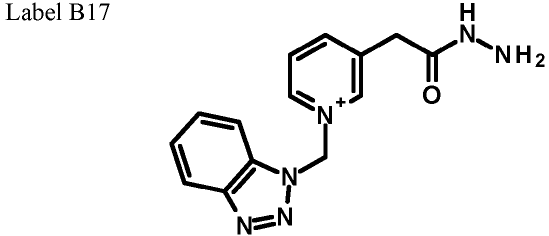 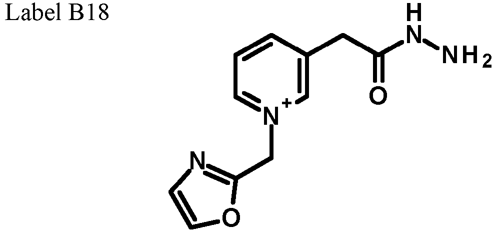 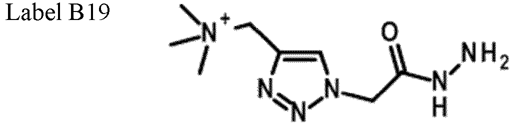 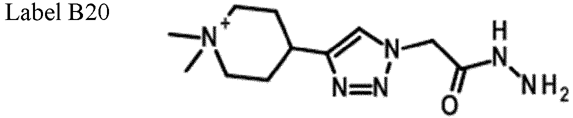 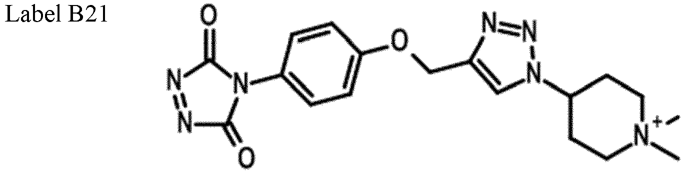 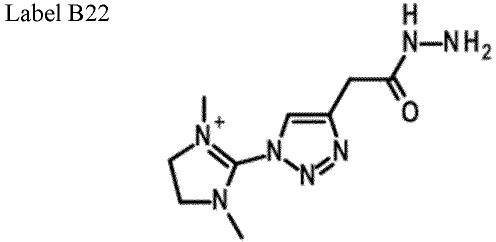 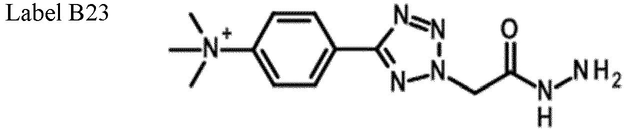 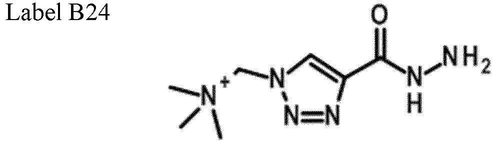 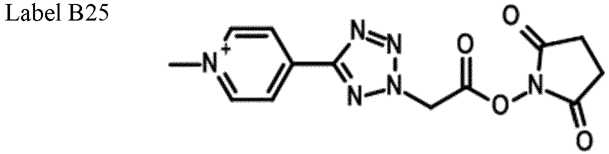 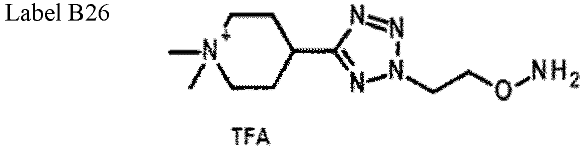 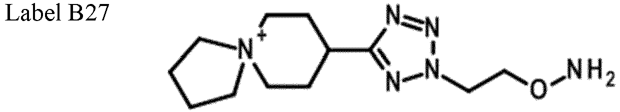 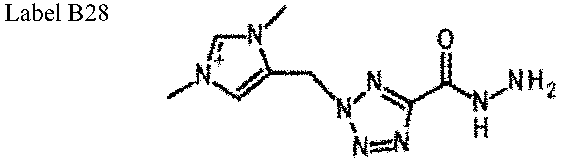 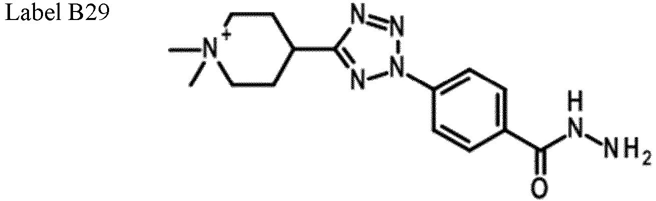 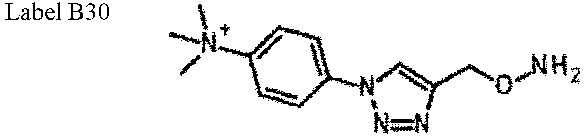 or combinations thereof.

The derivatization reagent may be selected from the group consisting of dansylchloride, carbamic acid, N-[2-[[[2-(diethylamino)ethyl]amino]carbonyl]-6-quinolinyl]-, 2,5-dioxo-1-pyrrolidinyl ester (RapiFluor-MS), 4-substituted 1,2,4-triazoline-3,5-diones (Cookson-type reagents), 4-phenyl-1,2,4-triazolin-3,5-dion-derivative (Amplifex Diene), 1-propanaminium, 3-(aminooxy)-N,N,N-trimethyl-compound comprising an appropriate counter ion (Amplifex Keto), acethydrazide trimethylammonium chloride (Girard T), 1-(carboxymethyl)pyridinium chloride hydrazide (Girard P) and pyridiyl amine.

At least one possible chemical structure of the derivatization reagent may be:

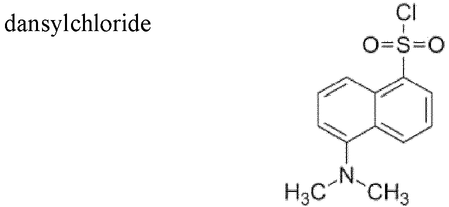

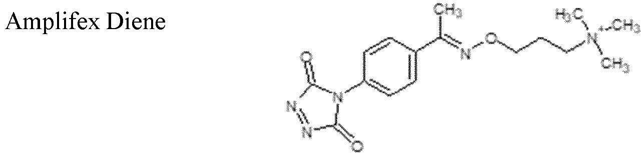

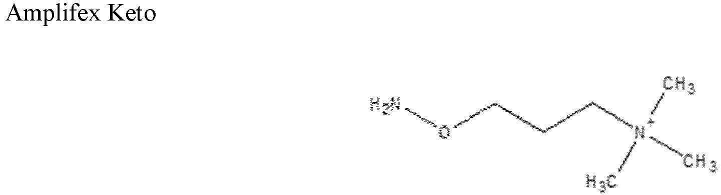

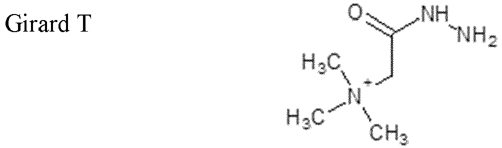

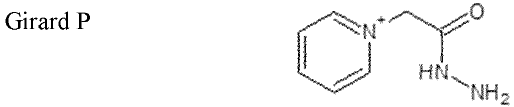

The method may comprise the compound of formula PI: 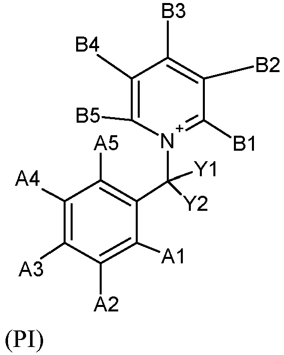
wherein one of the substituents B1, B2, B3, B4, B5 may be a coupling group Q, which is capable of forming a covalent bond with the analyte,
wherein the other substituents A1, A2, A3, A4, A5, B1, B2, B3, B4, B5 may be each independently selected from hydrogen, halogen, alkyl, N-acylamino, N,N-dialkylamino, alkoxy, thioalkoxy, hydroxy, cyano, alkoxycarbonyl, alkoxythiocarbonyl, acyl, nitro, thioacyl, aryloyl, fluoromethyl, difluoromethyl, trifluoromethyl, trifluoroethyl, cyanomethyl, cyanoethyl, hydroxyethyl, methoxyethyl, nitroethyl, acyloxy, aryloyloxy, cycloalkyl, aryl, heteroaryl, heterocycloalkyl, amino, isotope or derivative thereof,
wherein Y1 and Y2 may be each independently selected from hydrogen, methyl, ethyl, methoxy, substituted aromatic, unsubstituted aromatic, substituted cycloalkyl, unsubstituted cycloalkyl, substituted heteroaromatic, unsubstituted heteroaromatic, amine or wherein Y1 and Y2 may form a ring structure, which may be selected from substituted cycloalkyl, unsubstituted cycloalkyl, substituted aromatic, unsubstituted aromatic, substituted heteroaromatic, unsubstituted heteroaromatic.

A3 may be ammonium, B1 or B5 may be Q, wherein Q may be free of at least one atom, which is selected from O, N, S, Br. The term "Q is free of at least one atom, which is selected from O, N, S, Br" means here in the context of the disclosure, that Q does not comprise O or N or S or Br or combination thereof. In particular, Q is free of O, N, S and Br. In particular, Q is free of O, N, S and Br, if A3 is ammonium and B1 is Q. Alternativley, Q is free of O, N, S and Br, if A3 is ammonium and B5 is Q.

The derivatization reagent of formula PI may be selected from the following group: 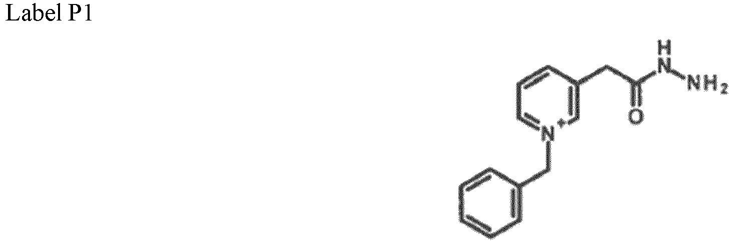 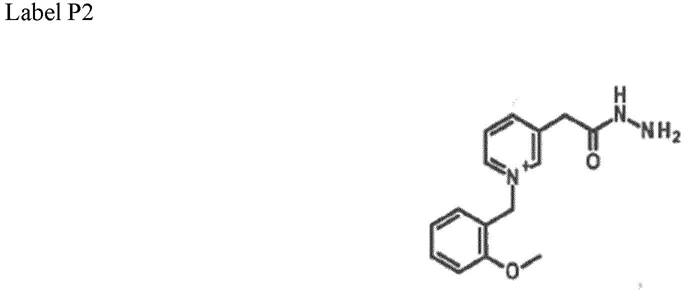 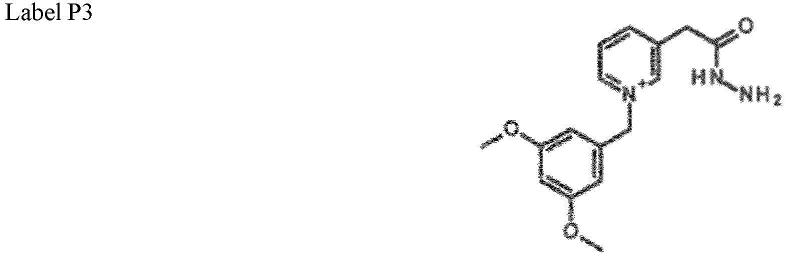 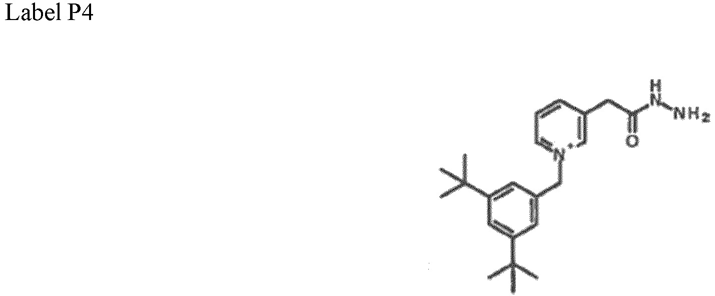 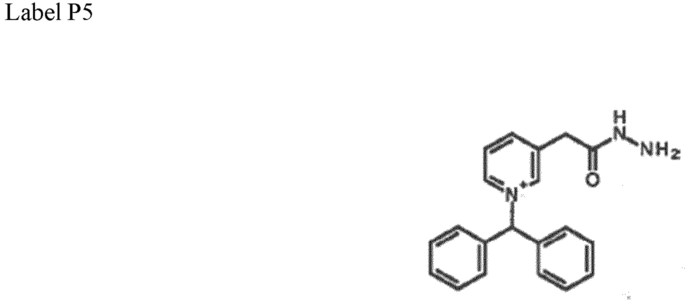 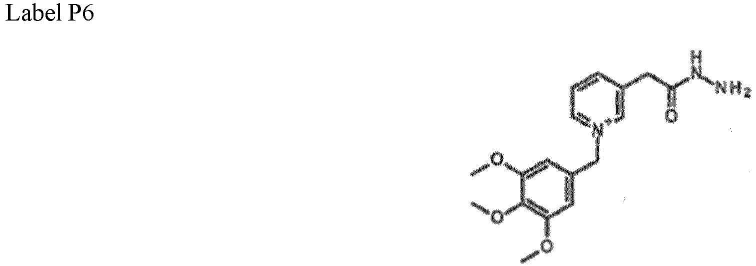 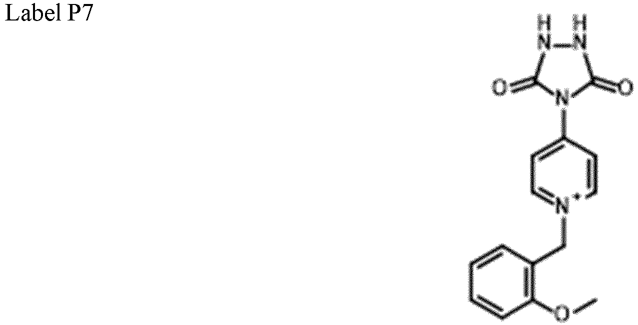 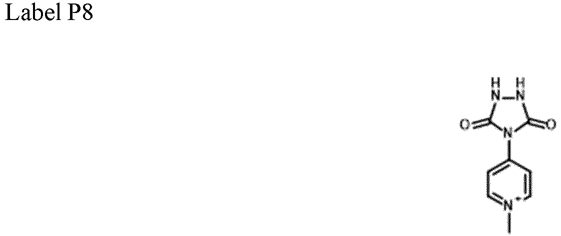 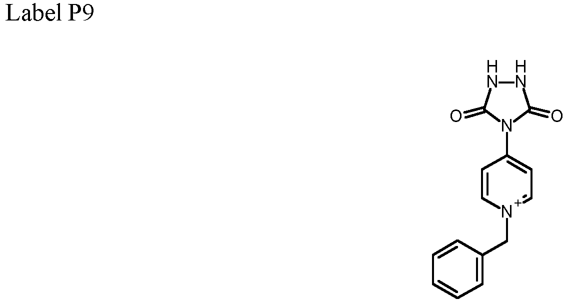 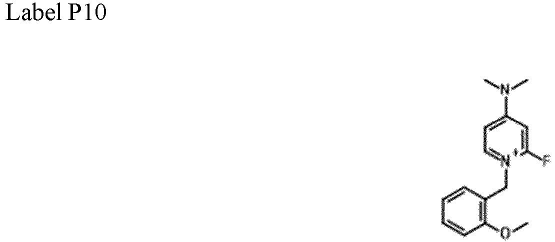 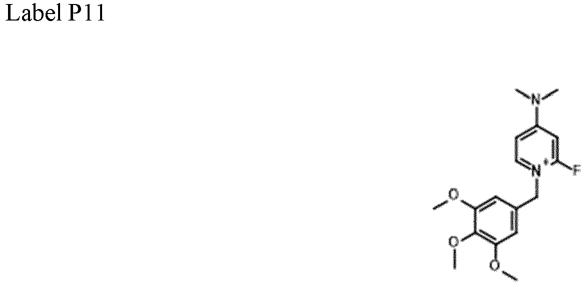 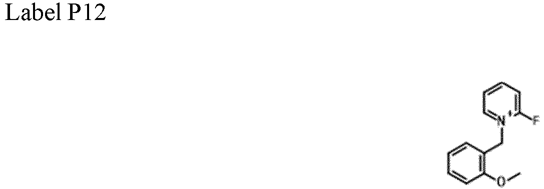 or combinations thereof.

The method may comprise the derivatization reagent of formula DI: 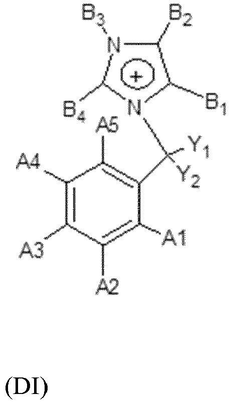
wherein one of the substituents B1, B2, B4 may be a coupling group Q, which may be capable of forming a covalent bond with the analyte,
wherein the other substituents A1, A2, A3, A4, A5, B1, B2, B4 may be each independently selected from hydrogen, halogen, alkyl, N- acylamino, N, N-dialkylamino, alkoxy, thioalkoxy, hydroxy, cyano, alkoxycarbonyl, alkoxythiocarbonyl, acyl, nitro, thioacyl, aryloyl, fluoromethyl, difluoromethyl, trifluoromethyl, trifluoroethyl, cyanomethyl, cyanoethyl, hydroxyethyl, methoxyethyl, nitroethyl, acyloxy, aryloyloxy, cycloalkyl, aryl, heteroaryl, heterocycloalkyl, amino, isotope or derivative thereof,
wherein B3 may be selected from alkyl, acetyl, vinyl, substituted aromatic, unsubstituted aromatic, substituted benzyl, unsubstituted benzyl, substituted cycloalkyl, unsubstituted cycloalkyl, isotope and derivative thereof,
wherein Y1 and Y2 may be each independently selected from hydrogen, methyl, ethyl, methoxy, substituted aromatic, unsubstituted aromatic, substituted cycloalkyl, unsubstituted cycloalkyl, substituted heteroaromatic, unsubstituted heteroaromatic, amine or wherein Y1 and Y2 may form a ring structure, which may be selected from substituted cycloalkyl, unsubstituted cycloalkyl, substituted aromatic, unsubstituted aromatic, substituted heteroaromatic, unsubstituted heteroaromatic.

The derivatization reagent of formula DI may be selected from the following group: 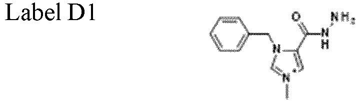 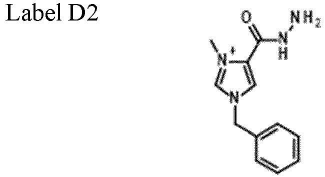 or combinations thereof.

The method may comprise derivatization reagents of formula CI: 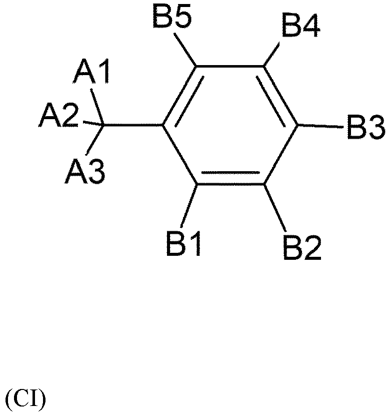
wherein one of the substituents B1, B2, B3, B4, B5 may be a coupling group Q, which may be capable of forming a covalent bond with the analyte,
wherein the other substituents A1, A2, B1, B2, B3, B4, B5 may be each independently selected from hydrogen, halogen, alkyl, modified alkyl, N-acylamino, N,N-dialkylamino, alkoxy, thioalkoxy, hydroxy, cyano, alkoxycarbonyl, alkoxythiocarbonyl, acyl, nitro, thioacyl, aryloyl, fluoromethyl, difluoromethyl, trifluoromethyl, trifluoroethyl, cyanomethyl, cyanoethyl, hydroxyethyl, methoxyethyl, nitroethyl, acyloxy, aryloyloxy, cycloalkyl, aryl, heteroaryl, heterocycloalkyl, amino, sulfur, isotope or derivative thereof,
wherein A3 may comprise ammonium, pyridinium, phosphonium or derivatives thereof,
wherein in case of A3 is ammonium and B1 or B5 is the coupling group Q, the coupling group Q may comprise a C atom, which may be separated by four single or double bonds from the C atom of the CA1A2A3 substituent and the coupling group Q may comprise a C-atom, which is separated by five single or double bonds from the C atom of the CA1A2A3 substituent.

The derivatization reagent of formula CI may be selected from the following group: 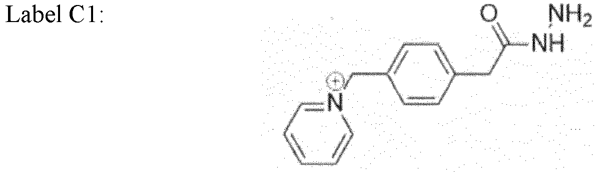 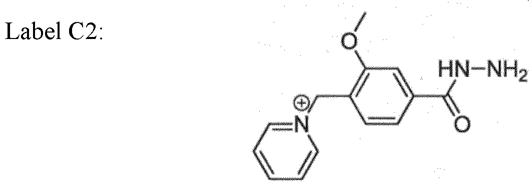 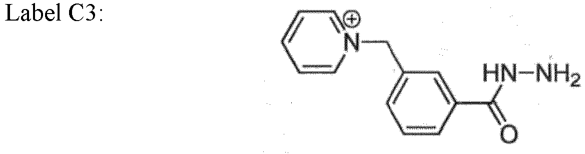 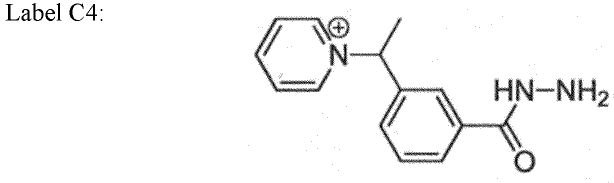 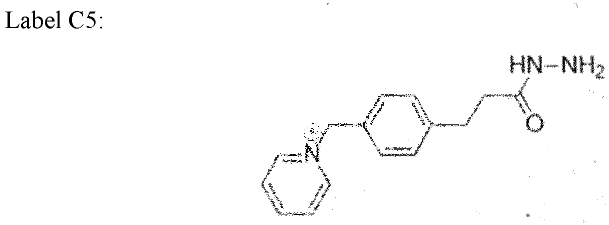 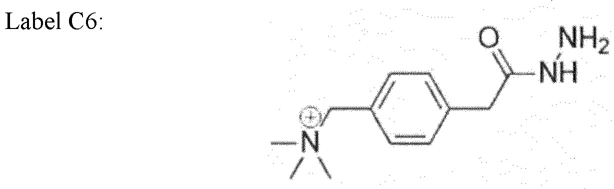 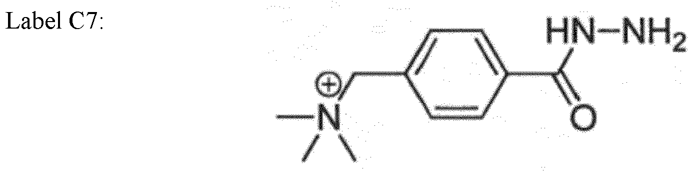 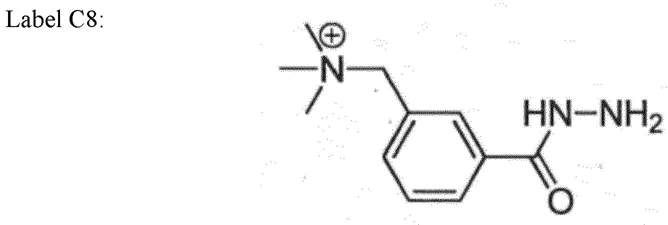 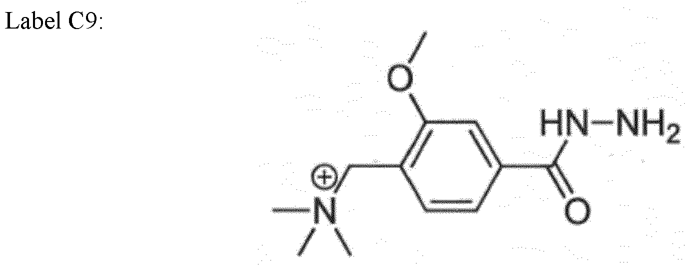 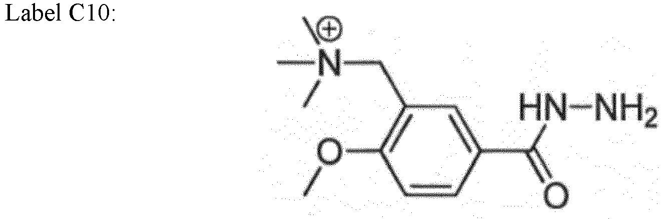 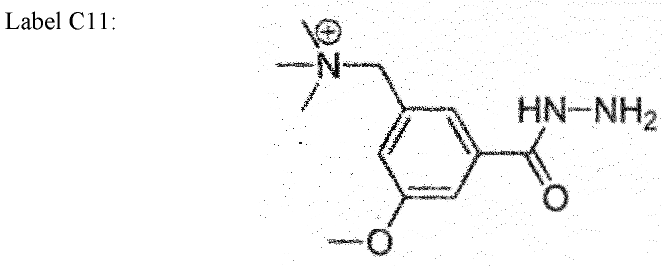 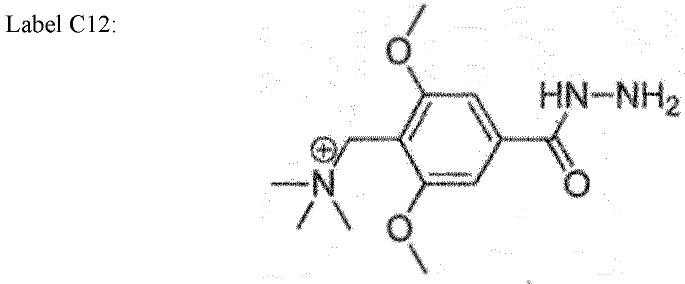 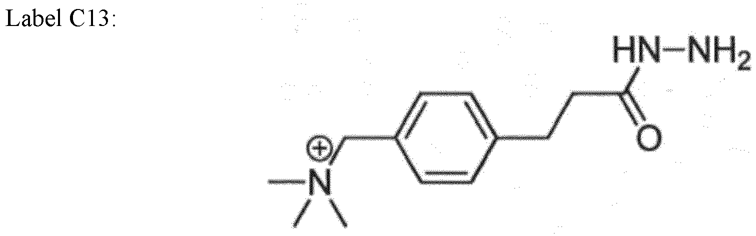 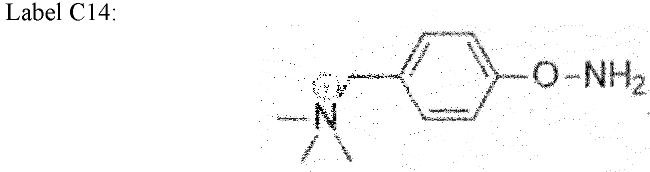 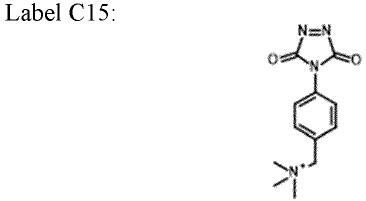 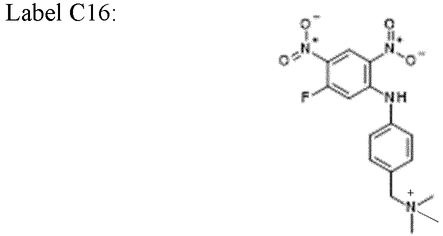 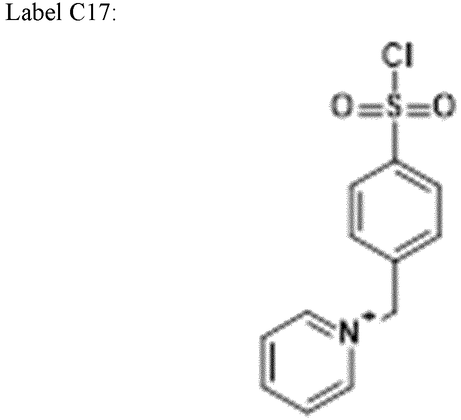 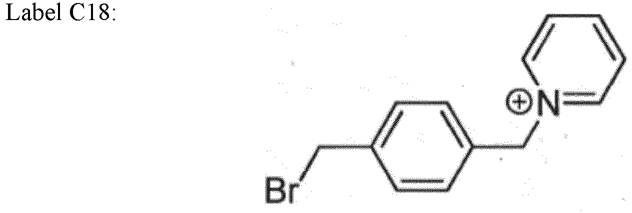 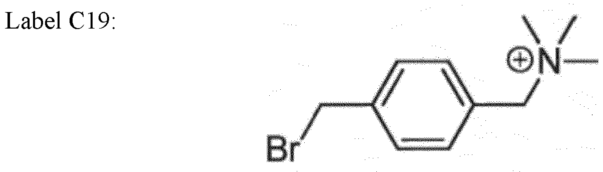 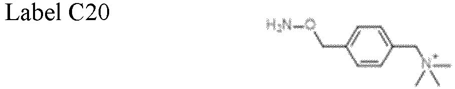 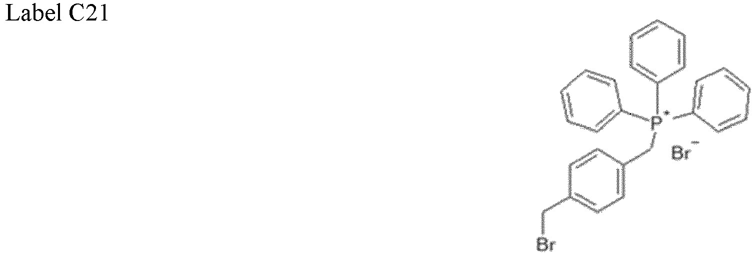 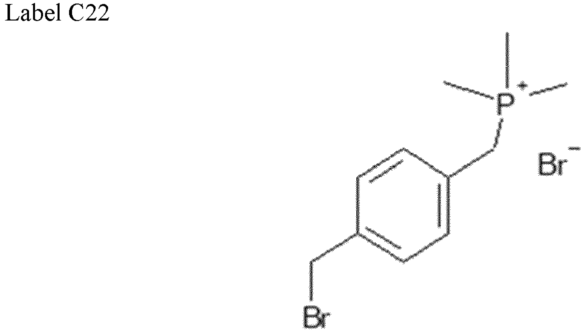 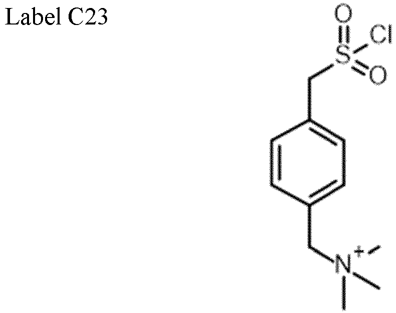 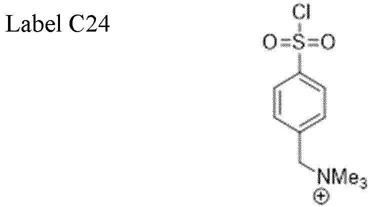 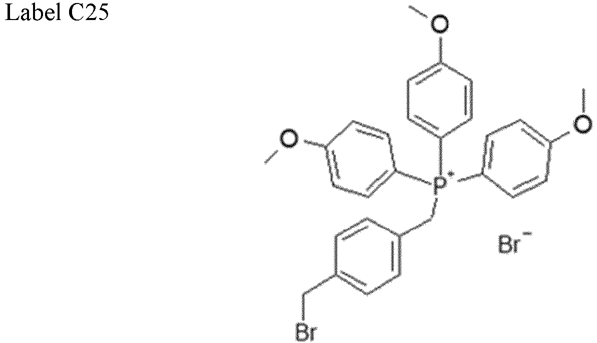 or combinations thereof.

The ratio of the analyte of interest to the compound may be in the range of 1:1 to 1:6.000.000 in step (b). In particular, the ratio of the analyte of interest to the compound may be in the range of 1:50000 to 1:100000 or 1:5000 to 1:10000 or 1:1 to 1:100 or 1:100 to 1:1000 or 1:1000000 to 1:2000000. The ratio depends on the kind of reaction, compound (derivatisation reagent), reaction kinetics, like reaction velocity, and/or temperature. The compound can be provided in an excess comparted to the analyte.

The analyte of interest may be selected from the group consisting of nucleic acid, amino acid, peptide, protein, metabolite, hormones, fatty acid, lipid, carbohydrate, steroid, ketosteroid, secosteroid, a molecule characteristic of a certain modification of another molecule, a substance that has been internalized by the organism, a metabolite of such a substance and combination thereof.

The analyte molecule may comprise a functional group selected from the group consisting of carbonyl group, diene group, hydroxyl group, amine group, imine group, ketone group, aldehyde group, thiol group, diol group, phenolic group, expoxid group, disulfide group, nucleobase group, carboxylic acid group, terminal cysteine group, terminal serine group and azide group, each of which is capable of forming a covalent bond with reactive unit K of compound. Further, it is also contemplated within the scope of the present invention that a functional group present on an analyte molecule would be first converted into another group that is more readily available for reaction with reactive unit K of compounds.

The analyte molecule may comprise a carbonyl group as functional group which may be selected from the group consisting of a carboxylic acid group, aldehyde group, keto group, a masked aldehyde, masked keto group, ester group, amide group, and anhydride group. Aldoses (aldehyde and keto) may exist as acetal and hemiacetals, a sort of masked form of the parent aldehyde/ keto.

The carbonyl group may be an amide group, the skilled person is well aware that the amide group as such is a stable group, but that it can be hydrolyzed to convert the amide group into an carboxylic acid group and an amino group. Hydrolysis of the amide group may be achieved via acid/base catalysed reaction or by enzymatic process either of which is well-known to the skilled person. The carbonyl group may be a masked aldehyde group or a masked keto group, the respective group may be either a hemiacetal group or acetal group, in particular a cyclic hemiacetal group or acetal group. The acetal group may be converted into an aldehyde or keto group before reaction with the compound.

The carbonyl group may be a keto group. The keto group may be transferred into an intermediate imine group before reacting with the reactive unit of compounds. The analyte molecule comprising one or more keto groups may be a ketosteroid. The ketosteroid may be selected from the group consisting of testosterone, epitestosterone, dihydrotestosterone (DHT), desoxymethyltestosterone (DMT), tetrahydrogestrinone (THG), aldosterone, estrone, 4-hydroxyestrone, 2-methoxyestrone, 2-hydroxyestrone, 16-ketoestradiol, 16-alpha-hydroxyestrone, 2-hydroxyestrone-3-methylether, prednisone, prednisolone, pregnenolone, progesterone, dehydroepiandrosterone (DHEA), 17-hydroxypregnenolone, 17-hydroxyprogesterone, androsterone, epiandrosterone, Δ4-androstenedione, 11-deoxycortisol, corticosterone, 21-deoxycortisol, 11-deoxycorticosterone, allopregnanolone and aldosterone.

The carbonyl group may be a carboxyl group. The carboxyl group may react directly with the compound or it may be converted into an activated ester group before reaction with the compound. The analyte molecule comprising one or more carboxyl groups may be selected from the group consisting of Δ8-tetrahydrocannabinolic acid, benzoylecgonin, salicylic acid, 2-hydroxybenzoic acid, gabapentin, pregabalin, valproic acid, vancomycin, methotrexate, mycophenolic acid, montelukast, repaglinide, furosemide, telmisartan, gemfibrozil, diclofenac, ibuprofen, indomethacin, zomepirac, isoxepac and penicillin. The analyte molecule comprising one or more carboxyl groups may be an amino acid selected from the group consisting of arginine, lysine, aspartic acid, glutamic acid, glutamine, asparagine, histidine, serine, threonine, tyrosine, cysteine, tryptophan, alanine, isoleucine, leucine, methionine, phenyalanine, valine, proline and glycine.

The carbonyl group may be an aldehyde group. The aldehyde group may be transferred into an intermediate imine group before reacting with the reactive unit of compounds. The analyte molecule comprising one or more aldehyde groups may be selected from the group consisting of pyridoxal, N-acetyl-D-glucosamine, alcaftadine, streptomycin and josamycin.

The carbonyl group may be an carbonyl ester group. The analyte molecule comprising one or more ester groups may be selected from the group consisting of cocaine, heroin, Ritalin, aceclofenac, acetylcholine, amcinonide, amiloxate, amylocaine, anileridine, aranidipine artesunate and pethidine.

The carbonyl group may be an anhydride group. The analyte molecule comprising one or more anhydride groups may be selected from the group consisting of cantharidin, succinic anhydride, trimellitic anhydride and maleic anhydride.

The analyte molecule may comprise one or more diene groups, in particular to conjugated diene groups, as functional group. The analyte molecule comprising one or more diene groups may be a secosteroid. The secosteroid may be selected from the group consisting of cholecalciferol (vitamin D3), ergocalciferol (vitamin D2), calcifediol, calcitriol, tachysterol, lumisterol and tacalcitol. In particular, the secosteroid may be vitamin D, in particular vitamin D2 or D3 or derivates thereof. The secosteroid may be selected from the group consisting of vitamin D2, vitamin D3, 25-hydroxyvitamin D2, 25-hydroxyvitamin D3 (calcifediol), 3-epi-25-hydroxyvitamin D2, 3-epi-25-hydroxyvitamin D3, 1,25-dihydroxyvitamin D2, 1,25-dihydroxyvitamin D3 (calcitriol), 24,25-dihydroxyvitamin D2, 24,25-dihydroxyvitamin D3. The analyte molecule comprising one or more diene groups may be selected from the group consisting of vitamin A, tretinoin, isotretinoin, alitretinoin, natamycin, sirolimus, amphotericin B, nystatin, everolimus, temsirolimus and fidaxomicin.

The analyte molecule may comprise one or more hydroxyl group as functional group. The analyte molecule may comprise a single hydroxyl group or two hydroxyl groups. In cases where more than one hydroxyl group is present, the two hydroxyl groups may be positioned adjacent to each other (1,2-diol) or may be separated by 1, 2 or 3 C atoms (1,3-diol, 1,4-diol, 1,5-diol, respectively). The analyte molecule may comprise a 1,2-diol group. In cases, where only one hydroxyl group is present, said analyte may be selected from the group consisting of primary alcohol, secondary alcohol and tertiary alcohol. In cases, where the analyte molecule comprises one or more hydroxyl groups, the analyte may be selected from the group consisting of benzyl alcohol, menthol, L-carnitine, pyridoxine, metronidazole, isosorbide mononitrate, guaifenesin, clavulanic acid, Miglitol, zalcitabine, isoprenaline, aciclovir, methocarbamol, tramadol, venlafaxine, atropine, clofedanol, alpha-hydroxyalprazolam, alpha-Hydroxytriazolam, lorazepam, oxazepam, Temazepam, ethyl glucuronide, ethylmorphine, morphine, morphine-3-glucuronide, buprenorphine, codeine, dihydrocodeine, p-hydroxypropoxyphene, O-desmethyltramadol, Desmetramadol, dihydroquinidine and quinidine. In cases, where the analyte molecule comprises more than one hydroxyl groups, the analyte may be selected from the group consisting of vitamin C, glucosamine, mannitol, tetrahydrobiopterin, cytarabine, azacitidine, ribavirin, floxuridine, Gemcitabine, Streptozotocin, adenosine, Vidarabine, cladribine, estriol, trifluridine, clofarabine, nadolol, zanamivir, lactulose, adenosine monophosphate, idoxuridine, regadenoson, lincomycin, clindamycin, Canagliflozin, tobramycin, netilmicin, kanamycin, ticagrelor, epirubicin, doxorubicin, arbekacin, streptomycin, ouabain, amikacin, neomycin, framycetin, paromomycin, erythromycin, clarithromycin, azithromycin, vindesine, digitoxin, digoxin, metrizamide, acetyldigitoxin, deslanoside, Fludarabine, clofarabine, gemcitabine, cytarabine, capecitabine, vidarabine, and plicamycin.

The analyte molecule may comprise one or more thiol group (including but not limited to alkyl thiol and aryl thiol groups) as functional group. The analyte molecule comprising one or more thiol groups may be selected from the group consisting of thiomandelic acid, DL-captopril, DL-thiorphan, N-acetylcysteine, D-penicillamine, glutathione, L-cysteine, zofenoprilat, tiopronin, dimercaprol, succimer.

The analyte molecule may comprise one or more disulfide group as functional group. The analyte molecule comprising one or more disulfide groups may be selected from the group consisting of glutathione disulfide, dipyrithione, selenium sulfide, disulfiram, lipoic acid, L-cystine, fursultiamine, octreotide, desmopressin, vapreotide, terlipressin, linaclotide and peginesatide. Selenium sulfide can be selenium disulfide, SeS₂, or selenium hexasulfide, Se₂S₆.

The analyte molecule may comprise one or more epoxide group as functional group. The analyte molecule comprising one or more epoxide groups may be selected from the group consisting of Carbamazepine-10,11-epoxide, carfilzomib, furosemide epoxide, fosfomycin, sevelamer hydrochloride, cerulenin, scopolamine, tiotropium, tiotropium bromide, methylscopolamine bromide, eplerenone, mupirocin, natamycin, and troleandomycin.

The analyte molecule may comprise one or more phenol groups as functional group. Analyte molecules comprising one or more phenol groups may be steroids or steroid-like compounds. The analyte molecule comprising one or more phenol groups may be a steroid or a steroid-like compound having an A-ring which is sp² hybridized and an OH group at the 3 -position of the A-ring. The steroid or steroid-like analyte molecule may be selected from the group consisting of estrogen, estrogen-like compounds, estrone (El), estradiol (E2), 17a-estradiol, 17b-estradiol, estriol (E3), 16-epiestriol, 17-epiestriol, and 16, 17-epiestriol and/or metabolites thereof. In embodiments, the metabolites are selected from the group consisiting of estriol, 16-epiestriol (16-epiE3), 17-epiestriol (17-epiE3), 16,17-epiestriol (16,17-epiE3), 16-ketoestradiol (16-ketoE2), 16a-hydroxyestrone (16a-OHEl), 2-methoxyestrone (2-MeOEl), 4-methoxyestrone (4-MeOEl), 2-hydroxyestrone-3-methyl ether (3-MeOEl), 2-methoxyestradiol (2-MeOE2), 4-methoxyestradiol (4-MeOE2), 2-hydroxyestrone (2-OHE1), 4-hydroxyestrone (4-OHE1), 2-hydroxyestradiol (2-OHE2), estrone (El), estrone sulfate (Els), 17a- estradiol (E2a), 17b-estradiol (E2B), estradiol sulfate (E2S), equilin (EQ), 17a-dihydroequilin (EQa), 17b-dihydroequilin (EQb), Equilenin (EN), 17-dihydroequilenin (ENa), 17α-dihydroequilenin, 17β-dihydroequilenin (ENb) , Δ8,9-dehydroestrone (dEl), Δ8,9-dehydroestrone sulfate (dEls), Δ9-tetrahydrocannabinol, mycophenolic acid. β or b can be used interchangeable. α and a can be used interchangeable.

The analyte molecule may comprise an amine group as functional group. The amine group may be an alkyl amine or an aryl amine group. The analyte comprising one or more amine groups may be selected from the group consisting of proteins and peptides. The analyte molecule comprising an amine group may be selected from the group consisting of 3,4-methylenedioxyamphetamine, 3,4-methylenedioxy-N-ethylamphetamine, 3,4-methylenedioxymethamphetamine, Amphetamine, Methamphetamine, N-methyl-1,3-benzodioxolylbutanamine, 7-aminoclonazepam, 7-aminoflunitrazepam, 3,4-dimethylmethcathinone, 3-fluoromethcathinone, 4-methoxymethcathinone, 4-methylethcathinone, 4-methylmethcathinone, amfepramone, butylone, ethcathinone, elephedrone, methcathinone, methylone, methylenedioxypyrovalerone, benzoylecgonine, dehydronorketamine, ketamine, norketamine, methadone, normethadone, 6-acetylmorphine, diacetylmorphine, morphine, norhydrocodone, oxycodone, oxymorphone, phencyclidine, norpropoxyphene, amitriptyline, clomipramine, dothiepin, doxepin, imipramine, nortriptyline, trimipramine, fentanyl, glycylxylidide, lidocaine, monoethylglycylxylidide, N-acetylprocainamide, procainamide, pregabalin, 2-Methylamino-1-(3,4-methylendioxyphenyl)butan, N-methyl-1,3-benzodioxolylbutanamine, 2-Amino-1-(3,4-methylendioxyphenyl)butan, 1,3-benzodioxolylbutanamine, normeperidine, O-Destramadol, desmetramadol, tramadol, lamotrigine, Theophylline, amikacin, gentamicin, tobramycin, vancomycin, Methotrexate, Gabapentin sisomicin and 5-methylcytosine.

The analyte molecule may be a carbohydrate or substance having a carbohydrate moiety, e.g. a glycoprotein or a nucleoside. The analyte molecule is a monosaccharide, in particular selected from the group consisting of ribose, desoxyribose, arabinose, ribulose, glucose, mannose, galactose, fucose, fructose, N-acetylglucosamine, N-acetylgalactosamine, neuraminic acid, N-acetylneurominic acid, etc.. The analyte molecule may be an oligosaccharide, in particular selected from the group consisting of a disaccharide, trisaccharid, tetrasaccharide, polysaccharide. The disaccharide may be selected from the group consisting of sucrose, maltose and lactose. In embodiments of the first aspect of the present invention, the analyte molecule is a substance comprising above described mono-, di-, tri-, tetra-, oligo- or polysaccharide moiety.

The analyte molecule may comprise an azide group as functional group which may be selected from the group consisting of alkyl or aryl azide. The analyte molecule comprising one or more azide groups may be selected from the group consisting of zidovudine and azidocillin.

Such analyte molecules may be present in biological or clinical samples such as body liquids, e.g. blood, serum, plasma, urine, saliva, spinal fluid, etc., tissue or cell extracts, etc. The analyte molecule(s) may be present in a biological or clinical sample selected from the group consisting of blood, serum, plasma, urine, saliva, spinal fluid, and a dried blood spot. The analyte molecules may be present in a sample which is a purified or partially purified sample, e.g. a purified or partially purified protein mixture or extract.

The reactive unit K may be selected from the group consisting of a carbonyl reactive unit, a diene reactive unit, a hydroxyl reactive unit, an amino reactive unit, an imine reactive unit, a thiol reactive unit, a diol reactive unit, a phenol reactive unit, an epoxide reactive unit, a disulfide reactive unit, and an azido reactive unit.

The reactive unit K may be a carbonyl reactive unit, which is capable of reacting with any type of molecule having a carbonyl group. The carbonyl reactive unit may be selected from the group consisting of carboxyl reactive unit, keto reactive unit, aldehyde reactive unit, anhydride reactive unit, carbonyl ester reactive unit, and imide reactive unit. The carbonyl-reactive unit may have either a super-nucleophilic N atom strengthened by the α-effect through an adjacent O or N atom NH₂-N/O or a dithiol molecule.

The carbonyl-reactive unit may be selected from the group consisting of
(i) a hydrazine unit, e.g. a H₂N-NH-, or H₂N-NR1- unit, wherein R1 is aryl or C1-4 alkyl, particularly C1 or C2 alkyl, optionally substituted,
(ii) a hydrazide unit, in particular a carbo-hydrazide or a sulfohydrazide, in particular a H₂N-NH-C(O)-, or H₂N-NR2-C(O)- unit, wherein R2 is aryl or C1-4 alkyl, particularly C1 or C2 alkyl, optionally substituted,
(iii) a hydroxylamino unit, e.g. a H₂N-O- unit, and
(iv) a dithiol unit, particularly a 1,2-dithiol or 1,3-dithiol unit.

In cases, where the carbonyl reactive unit is a carboxyl reactive unit, the carboxyl reactive units may react with carboxyl groups on an analyte molecule. The carboxyl reactive unit may be selected from the group consisting of a diazo unit, an alkylhalide, amine, and hydrazine unit.

Analyte molecules may comprise a ketone or aldehyde group and Q may be a carbonyl reactive unit, which is selected from the group:
(i) a hydrazine unit,
(ii) a hydrazide unit,
(iii) a hydroxylamino unit, and
(iv) a dithiol unit.

The reactive unit K may be a diene reactive unit, which is capable of reacting with an analyte comprising a diene group. The diene reactive unit may be selected from the group consisting of Cookson-type reagents, e.g. 1,2,4-triazoline-3,5-diones, which are capable to act as a dienophile.

The reactive unit K may be a hydroxyl reactive unit, which is capable of reacting with an analyte comprising a hydroxyl group. The hydroxyl reactive units may be selected from the group consisting of sulfonyl chlorides, activated carboxylic esters (NHS, or imidazolide), and fluoro aromates/ heteroaromates capable for nucleophilic substitution of the fluorine (T. Higashi J Steroid Biochem Mol Biol. 2016 Sep; 162:57-69). The reactive unit K may be a diol reactive unit which reacts with an diol group on an analyte molecule. In cases, where the reactive unit is a 1,2 diol reactive unit, the 1,2 diol reactive unit may comprise boronic acid. In further embodiments, diols can be oxidised to the respective ketones or aldehydes and then reacted with ketone/aldehyde-reactive unit(s) K.

The amino reactive unit may react with amino groups on an analyte molecule. The amino-reactive unit may be selected from the group consisting of active ester group such as N-hydroxy succinimide (NHS) ester or sulfo-NHS ester, pentafluoro phenyl ester, cabonylimidazole ester, quadratic acid esters, a hydroxybenzotriazole (HOBt) ester, 1-hydroxy-7-azabenzotriazole (HOAt) ester, and a sulfonylchloride unit.

The thiol reactive unit may react with a thiol group on an analyte molecule. The thiole reactive unit may be selected from the group consisting of haloacetyl group, in particular selected from the group consisting of Br/I-CH₂-C(=O)- unit, acrylamide/ester unit, unsaturated imide unit such as maleimide, methylsulfonyl phenyloxadiazole and sulfonylchloride unit.

The phenol reactive unit may react with phenol groups on an analyte molecule. The phenol-reactive unit may be selected from the group consisting of active ester unit such as N-hydroxy succinimide (NHS) ester or sulfo-NHS ester, pentafluoro phenyl ester, carbonylimidazole ester, quadratic acid esters, a hydroxybenzotriazole (HOBt) ester, 1-hydroxy-7-azabenzotriazole (HOAt) ester, and a sulfonylchloride unit. Phenol groups present on an analyte molecule can be reacted with highly reactive electrophiles like triazolinedione (like TAD) via a reaction (H. Ban et al J. Am. Chem. Soc., 2010, 132 (5), pp 1523-1525) or by diazotization or alternatively by ortho nitration followed by reduction to an amine which could then be reacted with an amine reactive reagent. The phenol-reactive unit may be fluoro-1-pyridinium.

The reactive unit K may be an epoxide reactive unit, which is capable of reacting with an analyte comprising a epoxide group. The epoxide reactive unit may be selected from the group consisting of amino, thiol, super-nucleophilic N atom strengthened by the α-effect through an adjacent O or N atom NH₂-N/O molecule.

The epoxide reactive unit may be selected from the group:
a hydrazine unit, e.g. a H₂N-NH-, or H₂N-NR¹- unit, wherein R¹ is aryl, aryl containing one or more heteroatoms or C₁₋₄ alkyl, particularly C₁ or C₂ alkyl, optionally substituted e.g. with halo, hydroxyl, and/or C₁₋₃ alkoxy,
a hydrazide unit, in particular a carbo-hydrazide or sulfo-hydrazide unit, in particular a H₂N-NH-C(O)-, or H₂N-NR²-C(O)- unit,
wherein R² is aryl, aryl containing one or more heteroatoms or C₁₋₄ alkyl, particularly C₁ or C₂ alkyl, optionally substituted e.g. with halo, hydroxyl, and/or C₁₋₃ alkoxy, and
a hydroxylamino unit, e.g. a H₂N-O- unit.

The reactive unit K may be a disulfide reactive unit, which is capable of reacting with an analyte comprising a disulfide group. The disulfide reactive unit may be selected from the group consisting of thiol. Disulfide group may be reduced to the respective thiol group and then reacted with thiol reactive units Q.

The reactive unit K may be a thiol-reactive group or may be an amino-reactive group such as an active ester group, e.g. N-hydroxysuccinimide (NHS) ester or sulpho-NHS ester, a hydroxybenzotrialzole (HOBt) ester or 1-hydroxy-7-acabenzotriazole (HOAt) ester group.

The reactive unit K may be selected from 4-substituted 1,2,4-triazolin-3,5-dione (TAD), 4-Phenyl-1,2,4-triazolin-3,5-dion (PTAD) or fluoro-substituted pyridinium.

The reactive unit K may be a azido reactive unit which reacts with azido groups on an analyte molecule. The azido-reactive unit may react with azido groups through azide-alkyne cycloaddition. The azido-reactive unit may be selected from the group consisting of alkyne (alkyl or aryl), linear alkyne or cyclic alkyne. The reaction between the azido and the alkyne can proceed with or without the use of a catalyst. The azido group can be reduced to the respective amino group and then reacted with amino reactive units K.

The functional group of the analyte may be selected from the options mentioned in the left coloumn of the table 1. The reactive group of Q of the corresponding functional group of the analyte may be selected from the the group mentioned in the right coloumn of table 1.

**Table 1: Functional group of the analyte and reactive groups for the specific labels**

| Functional group of the analyte | Reactive group |
|---|---|
| Amine | Active ester with NHS leaving group, pentafluorophenyl ester, squaric acid esters, sulfonyl chloride, |
| | ketone or aldehyde (reductive amination) |
| Thiol | Maleimide, iodoacetyl, methylsulfonyl phenyloxadiazole |
| Diol | Boronic acid (or oxidation to ketone or aldehyde) |
| Ketone, aldehyde | *O*-substituted hydroxylamine, hydrazines, hydrazides. |
| Diene | Dienophiles, triazolinedione (TAD) |
| Phenoles | Ene reaction triazolinedione (TAD), ortho nitration/reduction, diazo formation/nucleophilic substitution. |
| | Active ester with NHS leaving group, pentafluorophenyl ester, squaric acid esters, sulfonyl chloride, fluoro-1-pyridinium. |
| Nucleobase | Chloro acetyl/ Pt complexes |
| Unspecific | Azide (Nitrene) |
| Carboxylic acids | EDAC activation => amine |
| | Base / alkyl halide |
| | Chloroformate/ alcohol |
| | Diazoalkane |
| Terminal cysteine | Hetero aryl/Aryl cyanides |
| Terminal serine | Oxidation (followed by aldehyde reactive reagents) |

The analyte of interest may be a peptide, preferably selected from the group consisting of exhibiting free -NH2 or -COOH groups.

Step a), then b), then c) and then d) may be performed.

Surprisingly, it was found that a way of a cross spray configuration (i.e. intermixing the first and the second analytical flow stream for forming a mixture or a derivatized analyte of interest) of two ESI analytical flow streams to be used instead of a permanently used dopant addition in the eluent flow. The two ESI analytical flow streams meets in space in front of the ion source and exhibits the mode of action in terms of either chemical reactions or ion enhancement. Additonally, the ability of cross spray configuration can be used to make chemical reactions within the space range in front of the ESI source and the effect of DMSO or other dopands in the cross flow on peptides and small molecule polysaccharides or estradiol.

The use of the method is for determining the presence or the level of an analyte of interest in a sample. All embodiments mentioned for the method apply for the use thereof and vice versa.

In an example, the description relates to a diagnostic system for determining the presence or the level of an analyte of interest in a sample, comprising a first and a second electrospray ionization source and a mass spectrometric unit to carry out the method according to the first aspect of the invention. All embodiments mentioned for the first aspect of the invention and/or second aspect of the invention may apply for the example and vice versa.

The diagnostic system of the example maybe a clinical diagnostic system.

The first and/or a second electrospray ionization source of the example can be e.g. a chip-based electrospray ionization technology from company Advion. The second electrospray ionization source, e.g. the second ESI sprayer, which applies the respective dopand can be a ESI spray capillary or a second chip based system e.g. Advion. It combines the benefits of liquid chromatography, mass spectrometry, chip-based infusion, fraction collection, and direct surface analysis into one integrated ion source platform. Other known ESI sources are also possible. The ESI source is known for a skilled person and therefore not explained in detail.

The mass spectrometric unit of the example can be e.g. a triple quadrupole mass spectrometer or a linear ion trap mass spectrometer. A mass spectrometer is known for a skilled person and thus not explained in detail.

A "clinical diagnostics system" according to the example is a laboratory automated apparatus dedicated to the analysis of samples for in vitro diagnostics. The clinical diagnostics system may have different configurations according to the need and/or according to the desired laboratory workflow. Additional configurations may be obtained by coupling a plurality of apparatuses and/or modules together. A "module" is a work cell, typically smaller in size than the entire clinical diagnostics system, which has a dedicated function. This function can be analytical but can be also pre-analytical or post analytical or it can be an auxiliary function to any of the pre-analytical function, analytical function or post-analytical function. In particular, a module can be configured to cooperate with one or more other modules for carrying out dedicated tasks of a sample processing workflow, e.g. by performing one or more pre-analytical and/or analytical and/or post-analytical steps. In particular, the clinical diagnostics system can comprise one or more analytical apparatuses, designed to execute respective workflows that are optimized for certain types of analysis, e.g. clinical chemistry, immunochemistry, coagulation, hematology, liquid chromatography separation, mass spectrometry, etc. Thus the clinical diagnostic system may comprise one analytical apparatus or a combination of any of such analytical apparatuses with respective workflows, where pre-analytical and/or post analytical modules may be coupled to individual analytical apparatuses or be shared by a plurality of analytical apparatuses. In alternative pre-analytical and/or post-analytical functions may be performed by units integrated in an analytical apparatus. The clinical diagnostics system can comprise functional units such as liquid handling units for pipetting and/or pumping and/or mixing of samples and/or reagents and/or system fluids, and also functional units for sorting, storing, transporting, identifying, separating, detecting.

The clinical diagnostic system according to the example can comprise a sample preparation station for the automated preparation of samples comprising analytes of interest, optionally a liquid chromatography (LC) separation station comprising a plurality of LC channels and/or a sample preparation/LC interface for inputting prepared samples into any one of the LC channels. In particular, the clinical diagnostic system is free of a separation station, e.g. a LC-HPLC unit or HPLC unit.

The clinical diagnostic system according to the example can further comprise a controller programmed to assign samples to pre-defined sample preparation workflows each comprising a pre-defined sequence of sample preparation steps and requiring a pre-defined time for completion depending on the analytes of interest. The clinical diagnostic system can further comprise a mass spectrometer (MS) and an LC/MS interface for connecting the LC separation station to the mass spectrometer.

A "sample preparation station" according to the example can be a pre-analytical module coupled to one or more analytical apparatuses or a unit in an analytical apparatus designed to execute a series of sample processing steps aimed at removing or at least reducing interfering matrix components in a sample and/or enriching analytes of interest in a sample. Such processing steps may include any one or more of the following processing operations carried out on a sample or a plurality of samples, sequentially, in parallel or in a staggered manner: pipetting (aspirating and/or dispensing) fluids, pumping fluids, mixing with reagents, incubating at a certain temperature, heating or cooling, centrifuging, separating, filtering, sieving, drying, washing, resuspending, aliquoting, transferring, storing, etc.).

A "liquid chromatography (LC) separation station" according to the example is an analytical apparatus or module or a unit in an analytical apparatus designed to subject the prepared samples to chromatographic separation in order for example to separate analytes of interest from matrix components, e.g. remaining matrix components after sample preparation that may still interfere with a subsequent detection, e.g. a mass spectrometry detection, and/or in order to separate analytes of interest from each other in order to enable their individual detection. According to an embodiment, the LC separation station is an intermediate analytical apparatus or module or a unit in an analytical apparatus designed to prepare a sample for mass spectrometry and/or to transfer the prepared sample to a mass spectrometer. In particular, the LC separation station is a multi-channel LC station comprising a plurality of LC channels. Preferably, the clinical diagnostic system is free of the liquid chromatography (LC) separation station.

The clinical diagnostic system according to the example, e.g. the sample preparation station, may also comprise a buffer unit for receiving a plurality of samples before a new sample preparation start sequence is initiated, where the samples may be individually randomly accessible and the individual preparation of which may be initiated according to the sample preparation start sequence.

The clinical diagnostic system according to the example makes use of LC coupled to mass spectrometry more convenient and more reliable and therefore suitable for clinical diagnostics. In particular, high-throughput, e.g. up to 100 samples/hour or more with random access sample preparation and LC separation can be obtained while enabling online coupling to mass spectrometry. Moreover the process can be fully automated increasing the walk-away time and decreasing the level of skills required.

The diagnostic system of the example may be free of a baffle, preferably a rotable baffle, wherein the baffle is arranged between the first and the second electrospray ionization capillary/ ESI sprayer outlet. The baffle system in a commercially available instrument e.g. Waters or Micromass mass spectrometers are used for separating two- or more ion spray tips which applies an ESI spray continuous. By having the sprays continious flowing a lack phase is avoided which is advantouge for the intended use of appling two separated ESI sprays which are baffle separated to recalibrate the mass spectrometer. If a dopand spray setup is used it is not of interest wether or not the dopand appling esi-sprayer works continuously.

The the use of a dopand in the method described herein for enhancing the sensitivity of mass spectrometric detection of an analyte of interest comprises formula I:

R1-SO₂-R2 (I)

wherein R1, R2 are each independently selected from alkyl or aryl, wherein alkyl comprises one to six C-atoms, wherein aryl comprises one to six C-atoms. Preferably, the total number of carbon atoms (n) which is the sum of the carbon numbers of R1 and R2 (R1 and R2 can be aryl and alkyl) is maximum n=12. More preferably, sum of the carbon numbers of R1 and R2 (R1 and R2 can be aryl and/or alkyl) is maximum n= 11 or 10 or 9 or 8 or 7 or 6 or 5.

All embodiments mentioned for the method and/or the use of the method may apply for the use of the dopand and vice versa.

The use of the dopands can be used instead of DMSO (as reference), which can be used within an eluent stream or within the method described herein.

In the use of the dopand, alkyl may be selected from the group consisting of methyl, ethyl, butyl, pentyl-, hexyl and there are respective iso-alternatives as well as cyclo alternatives (e.g. iso-butyl or cyclohexyl).

In the use of the dopand, aryl may be a phenyl group.

In the use of the dopand, the dopand may be selected from a substituted sulfolane, an unsubstitued sulfolane, a substituted dibutylsulfone, an unsubstitued dibutylsulfone, a substituted diphenylsulfone or an unsubstitued diphenylsulfone.

In the use of the dopand, a substituted sulfolane may be selected from the group consisting of 2-phenyl-2,3,4,5-tetrahydrothiophene-S,S-dioxide (13557-28-3), 3-phenyltetrahydrothiophene 1,1-dioxide (16766-64-6), Sulfolane-2,2-5,5-d₄ (20627-71-8), 3,3,4,4-D4-Tetramethylensulfon (31124-58-0), 2-benzylthiolane 1,1-dioxide (101944-68-7), 2,5-divinylsulfolane (76286-66-3), 3-cyclohexyl-tetrahydrothiophene-1,1-dioxide (71053-08-2) and 2-vinylthiolane 1,1-dioxide (71411-39-7). The numbers in brackets indicate the possible CAS numbers.

In the use of the dopand, an unsubstituted sulfolane may be selected from pentamethylene sulfone (4988-33-4) or hexamethyl sulfone (6251-33-8). The numbers in brackets indicate the possible CAS numbers.

In the use of the dopand, a substituted dibutylsulfone may be selected from the group consisting of diisoamyl sulfone (2051-06-1), ethyl 3,3-dimethylbutyl sulfone (92098-95-8) and 3,3-dimethyl-1-(propane-1-sulfonyl)-butane (91391-45-6). The numbers in brackets indicate the possible CAS numbers.

In the use of the dopand, a unsubstituted dibutylsulfone may be dibutylsulfone.

In the use of the dopand, a substituted diphenylsulfone may be selected from the group consisting of phenylsulfone, (cyclopropylsulfonyl)benzene (17637-57-9), cyclohexyl phenyl sulfone (6947-57-5), (cyclohex-enylsulfonyl)benzene, 1-cyclopenten-1-yl phenyl sulfone (64740-90-5), (cyclopentylsulfonyl)benzene (14633-46-6), 1-(phenylsulfonyl)bicyclo[1.1.0]butane (80989-84-0), and ((cyclopropylmethyl)sulfonyl)benzene (69563-28-6). The numbers in brackets indicate the possible CAS numbers.

In the use of the dopand, an unsubstituted diphenylsulfone may be diphenylsulfone.

In the use of the dopand, the dopand may be selected from the following formulae II to IV:

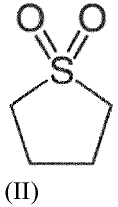

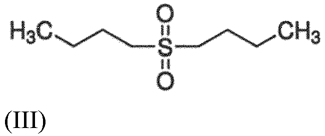

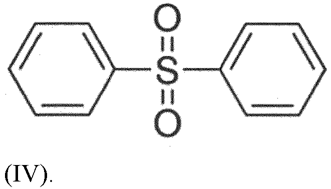

In the use of the dopand, the dopand may be free of a sulfoxide, e.g. dimethyl sulfoxide (DMSO).

### Examples

The following examples are provided to illustrate, but not to limit the presently claimed invention.

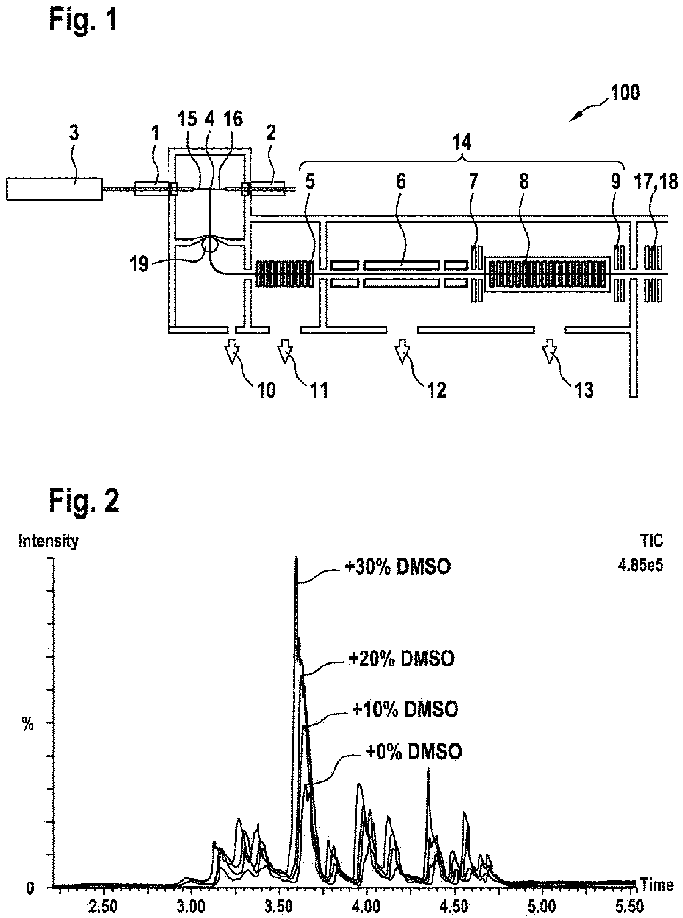

**Figure 1** shows a schematic view of a diagnostic system 100 according to the present invention. The diagnostic system 100 is used for determining the presence or the level of an analyte of interest in a sample, comprising a first and a second electrospray ionization source 1, 2 and a mass spectrometric unit 14, preferably to carry out the method according to the first aspect of the invention. The diagnostic system 100 is free of a baffle (not shown), where in normally the baffle would be arranged between the first and the second electrospray ionization source 1, 2. The baffle can be removed from commercial systems so that the two independent analytical flow streams 15, 16 can cross 4 there flow in the space in front of the mass spectrometric unit entrance. The first electrospray ionization source 1 is for a first analytical flow stream 15. The second electrospray ionization source is for a second analytical flow stream 16. The first analytical flow stream 15 comprises the analyte of interest. The analyte of interest and/or first analytical flow stream 15 can be purified by LC or HPLC, before the the first analytical flow stream 15 passes the first electrospray ionization source 1. The second analytical flow stream 16 comprises a dopand or a derivatization reagent, wherein each of the first analytical flow stream and the second analytical flow stream 15, 16 is comprised in gaseous form or aerosol. The first and the second analytical flow stream 15, 16 are crossed and/or intermixed and form a mixture or a derivatized analyte of interest. In case of a mixture, the mixture comprises the analyte of interest and the dopand. The presence or the level of an analyte of interest is determined in the mass spectrometric unit 14 using mass spectrometry.

**Figure 2** shows the TIC (Total Ion Count) in % vs. retension time of mixtures comprising peptides as analytes of interests and DMSO as a dopand in different concentrations (0%, 10%, 20% and 30% (v/v)). The peaks for the different chromatograms are normalized to the highest signal by keeping all other LC-MS parameters I (except the cross flow concentration of DMSO) constant. A LC-MS with standard gradient on a C18 column using water/acetonitrile as eluents used to separate an peptide test mixture from Sigma-Aldrich using the cross flow technique by adding in the cross flow different types of dopand concentrations ranging from 0-30% (v/v) of DMSO. Other suitable dopands can be used accordingly.

A signal enhancement can be observed by the addition of the dopand, e.g. DMSO. The signal intensity increases by increasing the concentration of the dopand, e.g. DMSO according to the method of the present invention. The amount of the analyte ot interest is constant at each injection. The separation by LC (peak width) are not influenced.

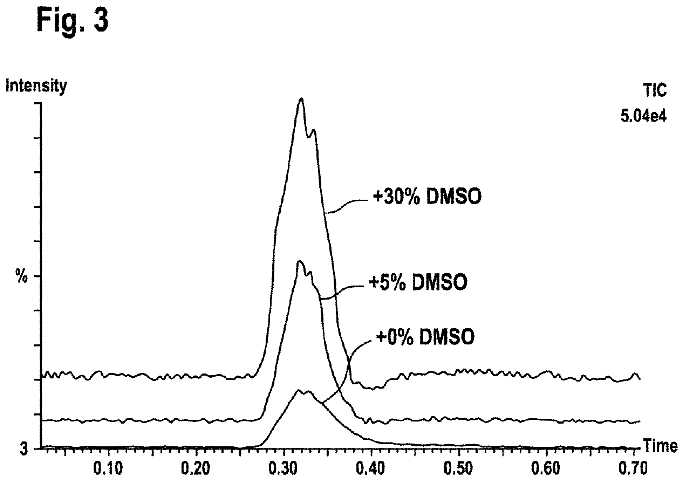

**Figure 3** shows the TIC in % vs. retension time of mixtures comprising toramycin as an analyte of interests and DMSO as a dopand in different concentrations (0%, 5% and 30% (v/v)) . Usage of the dopand spray effect for a small molecule based on poly saccharide with interest of therapeutics. In this case, tobramycin is used and further displayed that peak chromatographic peak widths are not changing by the cross flow technique.

A signal enhancement can be observed by the addition of the dopand, e.g. DMSO. The signal intensity increases by increasing the concentration of the dopand, e.g. DMSO according to the method of the present invention. The amount of the analyte ot interest is constant at each injection. The separation by LC (peak width) are not influenced. Other suitable dopands can be used accordingly.

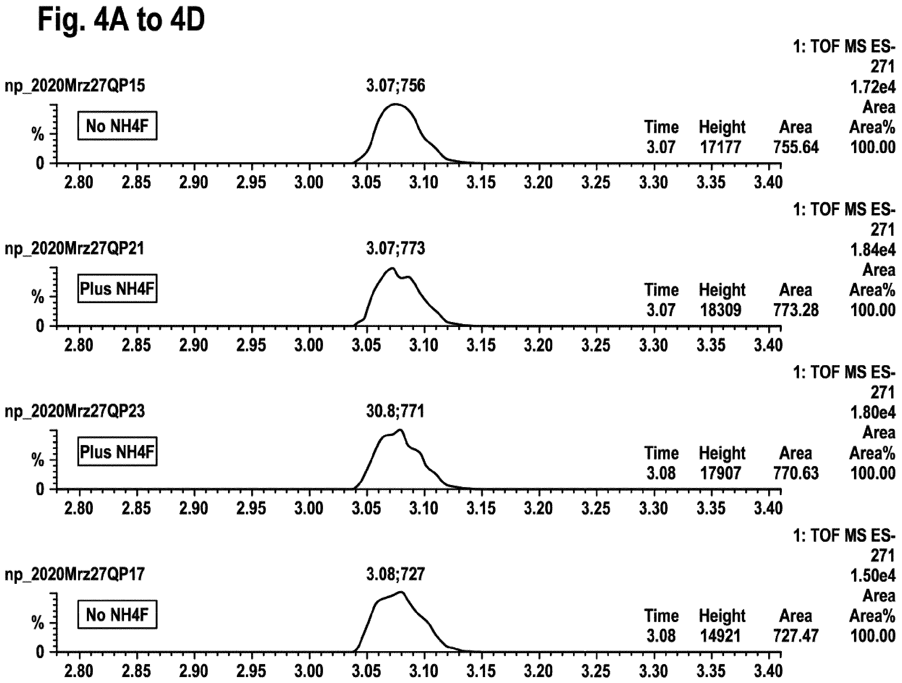

**Figures 4A to 4D** show the TIC in % vs. retension time of mixtures comprising estradiol as an analyte of interests with or without Ammoniumfluoride as a dopand (Figures 4A and 4D without NH₄F; Figures 4B and 4C with NH₄F). Usage of Ammoniumfluoride to enhance the [M-H]- Signal of estradiol by using the cross flow technique sccording to the method of the present invention. Fold change of about factor 1,04 (n=2, based on the peak area) as mean of two measurements against no addition of NH₄F was obtained.

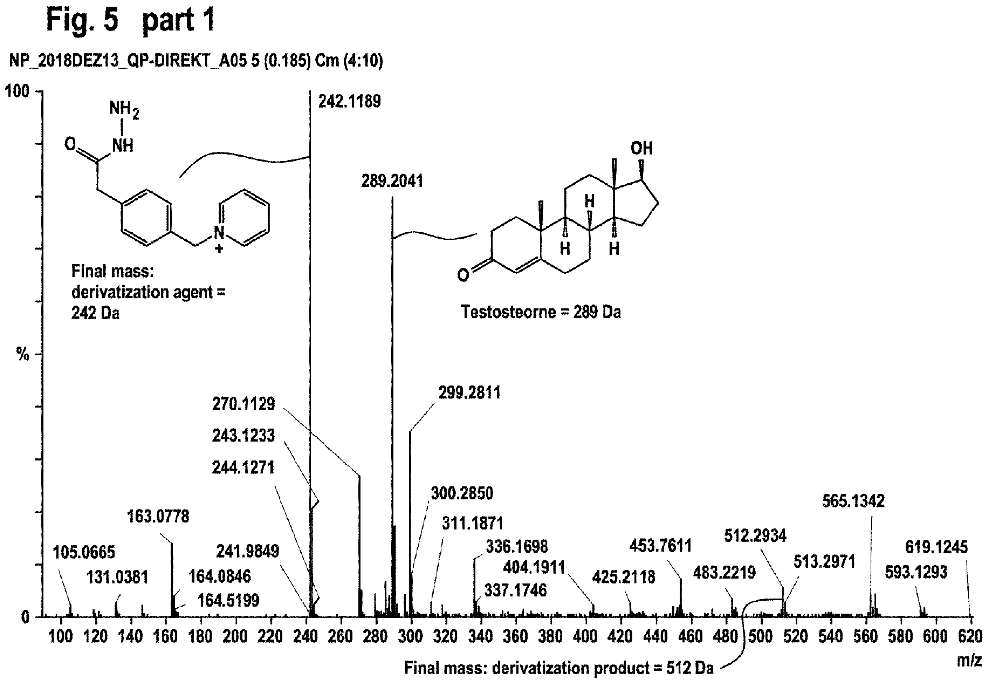

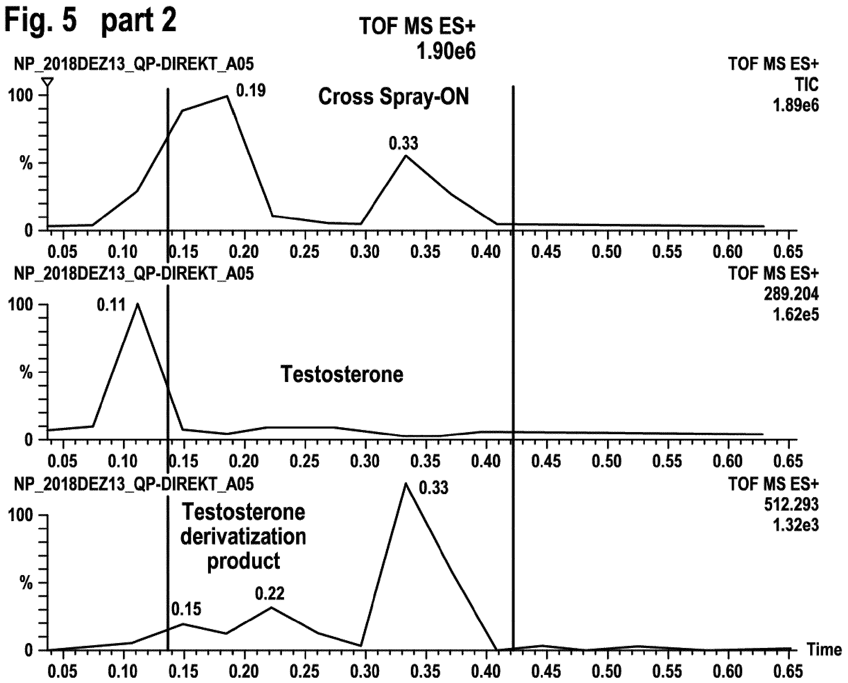

**Figure 5** shows the TIC in % vs. retension time of testosterone with an hydrazon derivatization reagent for coupling. Before turning on the cross spray (intermixing the analyte and the derivatization reagent) there is only testosterone visible, after turning on the cross spray the derivatization product can be seen. No backgournd signal can be detected in this mode, which means that no contamination of the analytical flow streams of the first and second electrospray ionization sources can be observed.

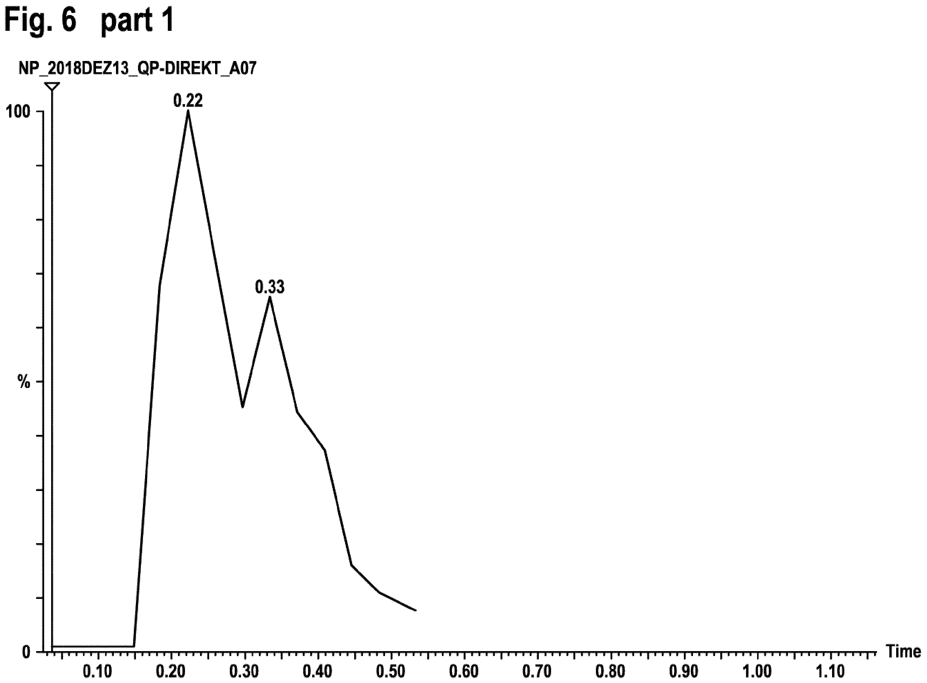

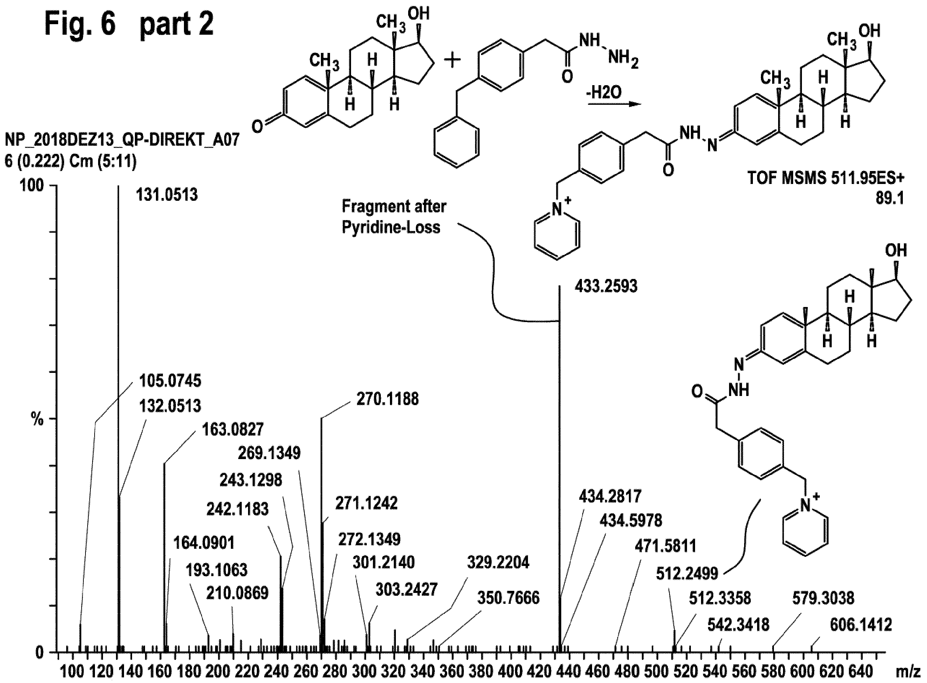

**Figure 6** shows the TIC in % vs. retension time of testosterone with a derivatization reagent. Confirmation of the reaction product from figure derivated testosterone-MZ2960 by MSMS in the cross flow spray configuration. The reaction of the derivatisation of testosterone was done by cross spray. The characteristic fragment 433 Da shows the pyridine loss of the testosterone - MZ2960 and is the prove of the succeeded derivatisation reaction in the cross spray.

The structure of MZ2960 is shown in Figure 6 and is as follows:

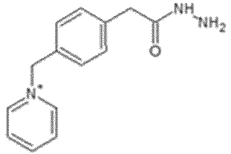

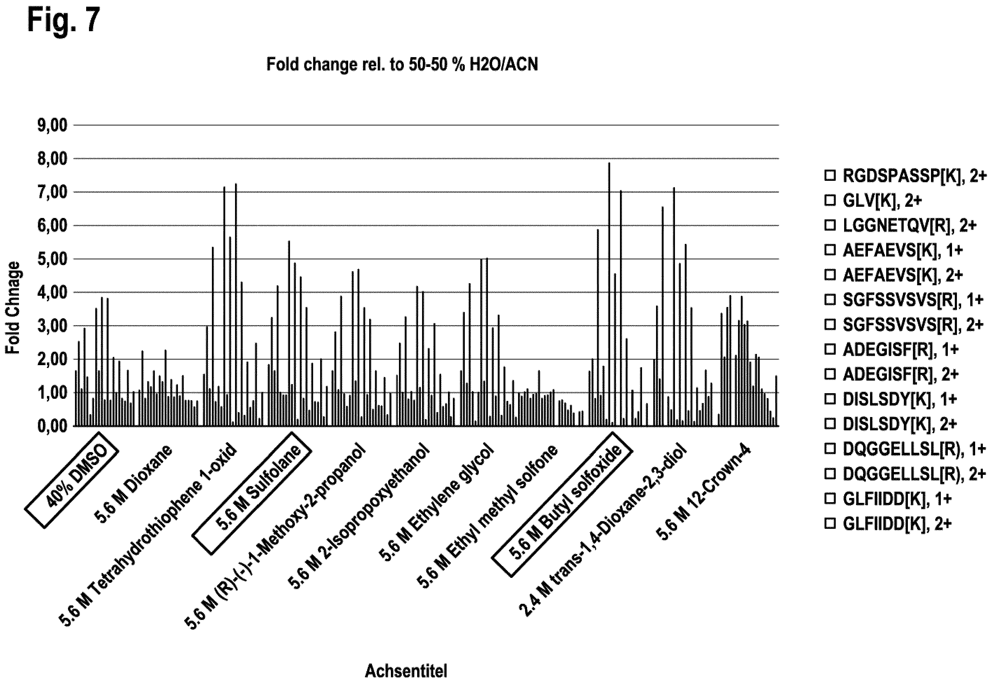

**Figure 7** shows the fold change of the peptide mixtures (purchased by Sigma Aldrich) respective peptides against a cross flow eluent of the blank H₂O/CAN (CAN = acetonitrile). The peptide mixtures are injected by HPLC via the first electrospray ionization source, while the dopand is sprayed by the second electrospray ionization source. As can be seen, the novel dopands, preferably sulfolane derived substances with phenylic and aliphatic structure elements ranging C numbers of C=1 to C=6, show advantages in signal enhancement. The signal enhancement effect of the dopands is also seen, when the dopand is mixed bevor the mass spectrometer.

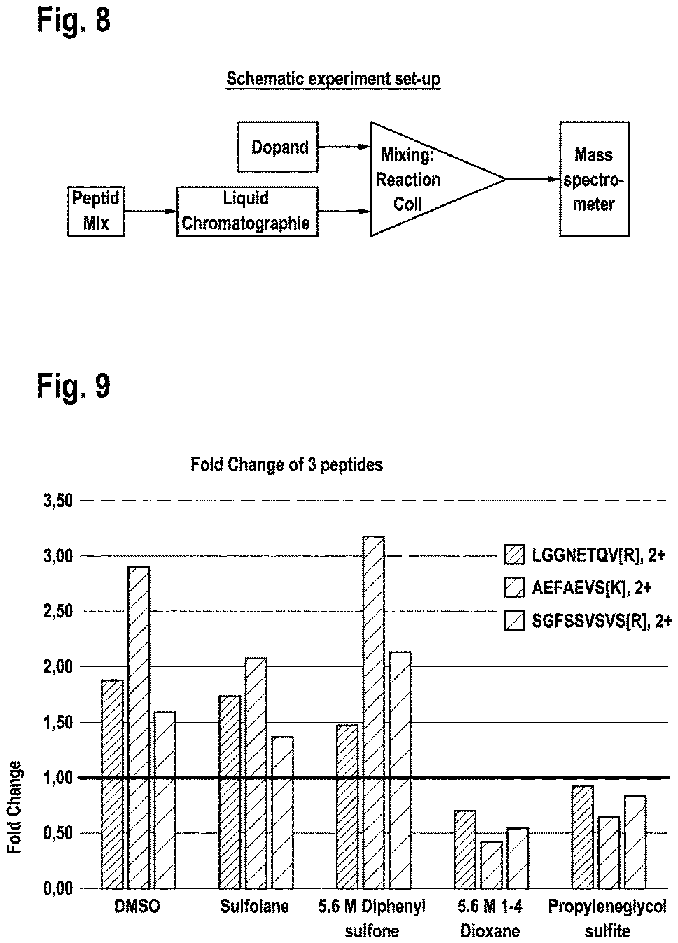

In **Figure 8** a possible schematic experiment set-up is shown. The peptid test mixture is mixed with the dopand after the column and before the mass spectrometer in a reaction coil. The results are shown in Figure 9.

**Figure 9** shows the fold change of three peptides as analytes of interest by the addition of different dopands like DMSO, sulfolane, diphenylsulfone, 1,4 dioxane and propyleneglycolsulfite. A LC-MS with standard gradient on a C18 column using water/acetonitrile as eluents used to separate an peptide test mixture purchased from Sigma-Aldrich. After the column and before the ESI-MS the dopand (DMSO, sulfolane, diphenylsulfone, dioxane, propyleneglycol sulfite) was added and mixed with the eluent by using a reaction coil. The plot shows the fold changes of three peptides by using different dopands. DMSO, sulfolane and diphenylsulfone push the signal of the peptides and dioxane and propyleneglycol shows no effect of signal enhancement. Therefore, the use of the disclosed dopands shows advantages compared to the prior art.

This patent application claims the priority of the European patent application 20211558.0.

## Claims

1. A method for determining the presence or the level of an analyte of interest in a sample comprising the following steps:
a) Providing a first electrospray ionization source for a first analytical flow stream comprising the analyte of interest,
b) Providing a second electrospray ionization source for a second analytical flow stream comprising a dopand or a derivatization reagent, wherein each of the first analytical flow stream and the second analytical flow stream is comprised in gaseous form or aerosol,
c) Intermixing the first and the second analytical flow stream for forming a mixture or a derivatized analyte of interest, wherein the mixture comprises the analyte of interest and the dopand, and
d) Determining the presence or the level of an analyte of interest in the sample using mass spectrometry.

2. The method of claim 1, wherein the method is performed for enhancing the mass spectrometric signal of the analyte of interest.

3. The method of claim 1 or 2, wherein the first analytical flow stream is intermixed with the second analytical flow stream after leaving the first and the second electrospray ionization source.

4. The method of any of the proceeding claims, wherein the first electrospray ionization source and the second electrospray ionization source are arranged at least 10° to 180° according a plan view over the two electrospray ionization sources and at least 10° to 180° according to a front view for the electrospray ionization sources.

5. The method of any of the proceeding claims, wherein the dopand is DMSO or NH₄F.

6. The method of any of the proceeding claims, wherein the second flow stream comprises a concentration of the dopand or a concentration of the derivatization reagent in the range of 5 to 35 % (v/v).

7. The method of any of the proceeding claims, wherein the derivatization reagent is selected from the group consisting of dansylchloride, carbamic acid, N-[2-[[[2-(diethylamino)ethyl]amino]carbonyl]-6-quinolinyl]-, 2,5-dioxo-1-pyrrolidinyl ester (RapiFluor-MS), 4-substituted 1,2,4-triazoline-3,5-diones (Cookson-type reagents), 4-phenyl-1,2,4-triazolin-3,5-dion-derivative (Amplifex Diene), 1-propanaminium, 3-(aminooxy)-N,N,N-trimethyl-compound comprising an appropriate counter ion (Amplifex Keto), acethydrazide trimethylammonium chloride (Girard T), 1-(carboxymethyl)pyridinium chloride hydrazide (Girard P) and pyridiyl amine.

8. The method of any of the proceeding claims, wherein the derivatization reagent comprises a compound of formula A or B: 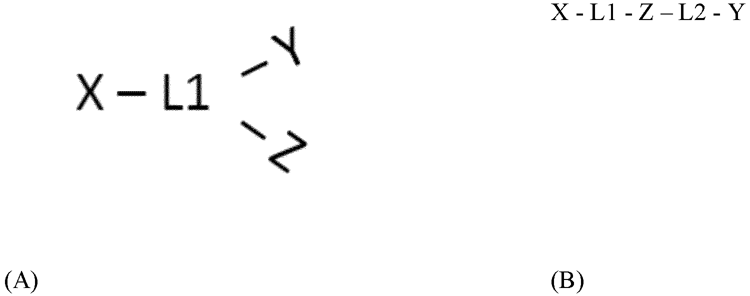 wherein
X is a reactive unit, which is in particular capable of forming a covalent bond with an analyte of interest,
L1 and L2 are independently of each other substituted or unsubstituted linker, in particular branched or linear linker,
Y is a neutral loss unit, and
Z is a charged unit comprising at least one permanently charged moiety, in particular comprising one permanently charged moiety,
including any salt thereof, or
wherein the derivatization reagent comprises a compound of formula PI: 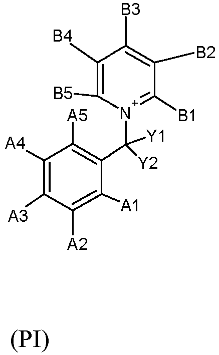
wherein one of the substituents B1, B2, B3, B4, B5 is a coupling group Q, which is capable of forming a covalent bond with the analyte,
wherein the other substituents A1, A2, A3, A4, A5, B1, B2, B3, B4, B5 are each independently selected from hydrogen, halogen, alkyl, N-acylamino, N,N-dialkylamino, alkoxy, thioalkoxy, hydroxy, cyano, alkoxycarbonyl, alkoxythiocarbonyl, acyl, nitro, thioacyl, aryloyl, fluoromethyl, difluoromethyl, trifluoromethyl, trifluoroethyl, cyanomethyl, cyanoethyl, hydroxyethyl, methoxyethyl, nitroethyl, acyloxy, aryloyloxy, cycloalkyl, aryl, heteroaryl, heterocycloalkyl, amino, isotope or derivative thereof,
wherein Y1 and Y2 are each independently selected from hydrogen, methyl, ethyl, methoxy, substituted aromatic, unsubstituted aromatic, substituted cycloalkyl, unsubstituted cycloalkyl, substituted heteroaromatic, unsubstituted heteroaromatic, amine or wherein Y1 and Y2 form a ring structure, which is selected from substituted cycloalkyl, unsubstituted cycloalkyl, substituted aromatic, unsubstituted aromatic, substituted heteroaromatic, unsubstituted heteroaromatic, or
wherein the derivatization reagent comprises a compound of formula DI: 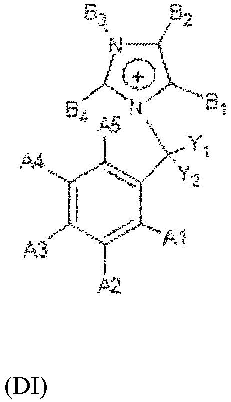
wherein one of the substituents B1, B2, B4 is a coupling group Q, which is capable of forming a covalent bond with the analyte,
wherein the other substituents A1, A2, A3, A4, A5, B1, B2, B4 are each independently selected from hydrogen, halogen, alkyl, N-acylamino, N,N-dialkylamino, alkoxy, thioalkoxy, hydroxy, cyano, alkoxycarbonyl, alkoxythiocarbonyl, acyl, nitro, thioacyl, aryloyl, fluoromethyl, difluoromethyl, trifluoromethyl, trifluoroethyl, cyanomethyl, cyanoethyl, hydroxyethyl, methoxyethyl, nitroethyl, acyloxy, aryloyloxy, cycloalkyl, aryl, heteroaryl, heterocycloalkyl, amino, isotope or derivative thereof,
wherein B3 is selected from alkyl, acetyl, vinyl, substituted aromatic, unsubstituted aromatic, substituted benzyl, unsubstituted benzyl, substituted cycloalkyl, unsubstituted cycloalkyl, isotope and derivative thereof,
wherein Y1 and Y2 are each independently selected from hydrogen, methyl, ethyl, methoxy, substituted aromatic, unsubstituted aromatic, substituted cycloalkyl, unsubstituted cycloalkyl, substituted heteroaromatic, unsubstituted heteroaromatic, amine or wherein Y1 and Y2 form a ring structure, which is selected from substituted cycloalkyl, unsubstituted cycloalkyl, substituted aromatic, unsubstituted aromatic, substituted heteroaromatic, unsubstituted heteroaromatic, or
wherein the derivatization reagent comprises a compound of formula CI: 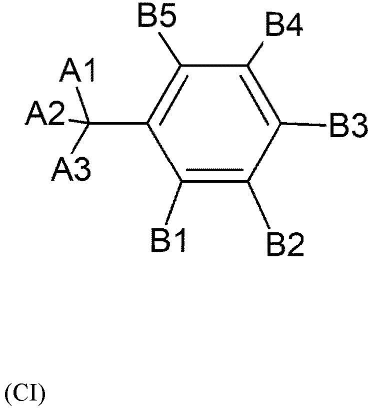
wherein one of the substituents B1, B2, B3, B4, B5 is a coupling group Q, which is capable of forming a covalent bond with the analyte,
wherein the other substituents A1, A2, B1, B2, B3, B4, B5 are each independently selected from hydrogen, halogen, alkyl, modified alkyl, N-acylamino, N,N-dialkylamino, alkoxy, thioalkoxy, hydroxy, cyano, alkoxycarbonyl, alkoxythiocarbonyl, acyl, nitro, thioacyl, aryloyl, fluoromethyl, difluoromethyl, trifluoromethyl, trifluoroethyl, cyanomethyl, cyanoethyl, hydroxyethyl, methoxyethyl, nitroethyl, acyloxy, aryloyloxy, cycloalkyl, aryl, heteroaryl, heterocycloalkyl, amino, sulfur, isotope or derivative thereof,
wherein A3 comprises ammonium, pyridinium, phosphonium or derivatives thereof,
wherein in case of A3 is ammonium and B1 or B5 is the coupling group Q, the coupling group Q comprises a C atom, which is separated by four single or double bonds from the C atom of the CA1A2A3 substituent and the coupling group Q comprises a C-atom, which is separated by five single or double bonds from the C atom of the CA1A2A3 substituent.

9. The method of any of the proceeding claims, wherein the analyte of interest is selected from the group consisting of nucleic acid, amino acid, peptide, protein, metabolite, hormones, fatty acid, lipid, carbohydrate, steroid, ketosteroid, secosteroid, a molecule characteristic of a certain modification of another molecule, a substance that has been internalized by the organism, a metabolite of such a substance and combination thereof, preferably wherein the analyte of interest is a peptide selected from the group consisting of exhibiting free -NH₂ or -COOH groups.

10. A use of the method of any one of claims 1 to 9 for determining the presence or the level of an analyte of interest in a sample.

11. A use of a dopand in the method according to any one of 1 to 9, wherein the dopand for enhancing the sensitivity of mass spectrometric detection of an analyte of interest comprises formula I:
R1-SO₂-R2 (I)
wherein R1, R2 are each independently selected from alkyl or aryl, wherein alkyl comprises one to six C-atoms, wherein aryl comprises one to six C-atoms.

12. The use of the dopand of claim 11, wherein the dopand is selected from a substituted sulfolane, an unsubstitued sulfolane, a substituted dibutylsulfone, an unsubstitued dibutylsulfone, a substituted diphenylsulfone or an unsubstitued diphenylsulfone.

13. The use of the dopand of any one of claims 11 to 12, wherein the dopand is free of sulfoxide, preferably free of DMSO.

## Patentansprüche

1. Verfahren zum Bestimmen der Gegenwart oder des Gehalts eines Analyten von Interesse in einer Probe, umfassend die folgenden Schritte:
a) Bereitstellen einer ersten Elektrosprayionisationsquelle für einen ersten Analysenstrom, der den Analyten von Interesse umfasst,
b) Bereitstellen einer zweiten Elektrosprayionisationsquelle für einen zweiten Analysenstrom, der einen Dotierstoff oder ein Derivatisierungsreagens umfasst, wobei jeder von dem ersten Analysenstrom und dem zweiten Analysenstrom in Gasform oder als Aerosol vorliegt,
c) Vermischen des ersten und des zweiten Analysenstromes zum Bilden eines Gemisches oder eines derivatisierten Analyten von Interesse, wobei das Gemisch den Analyten von Interesse und den Dotierstoff umfasst, und
d) Bestimmen der Gegenwart oder des Gehalts eines Analyten von Interesse in der Probe unter Anwendung von Massenspektrometrie.

2. Verfahren nach Anspruch 1, wobei das Verfahren zum Verstärken des massenspektrometrischen Signals des Analyten von Interesse durchgeführt wird.

3. Verfahren nach Anspruch 1 oder 2, wobei der erste Analysenstrom mit dem zweiten Analysenstrom vermischt wird, nachdem er die erste und die zweite Elektrosprayionisationsquelle verlassen hat.

4. Verfahren nach einem der vorstehenden Ansprüche, wobei die erste Elektrosprayionisationsquelle und die zweite Elektrosprayionisationsquelle mindestens 10° bis 180° gemäß einer Draufsicht über die beiden Elektrosprayionisationsquellen und mindestens 10° bis 180° gemäß einer Vorderansicht für die Elektrosprayionisationsquellen angeordnet sind.

5. Verfahren nach einem der vorstehenden Ansprüche, wobei der Dotierstoff DMSO oder NH₄F ist.

6. Verfahren nach einem der vorstehenden Ansprüche, wobei der zweite Strom eine Konzentration des Dotierstoffes oder eine Konzentration des Derivatisierungsreagens im Bereich von 5 bis 35 Vol.-% umfasst.

7. Verfahren nach einem der vorstehenden Ansprüche, wobei das Derivatisierungsreagens aus der Gruppe ausgewählt ist, die aus Dansylchlorid, Carbamidsäure, N-[2-[[[2-(Diethylamino)ethyl]amino]carbonyl]-6-chinolinyl]-2,5-Dioxo-1-pyrrolidinylester (RapiFluor-MS), 4-substituierten 1,2,4-Triazolin-3,5-dionen (Cookson-Reagenzien), 4-Phenyl-1,2,4-triazolin-3,5-dion-Derivat (Amplifex-Dien), 1-Propanaminium, 3-(Aminooxy)-N,N,N-trimethyl-Verbindung, umfassend ein entsprechendes Gegenion (Amplifex-Keto), Acethydrazidtrimethylammoniumchlorid (Girard T), 1-(Carboxymethyl)pyridiniumchloridhydrazid (Girard P) und Pyridiylamin besteht.

8. Verfahren nach einem der vorstehenden Ansprüche, wobei das Derivatisierungsreagens eine Verbindung der Formel A oder B umfasst: 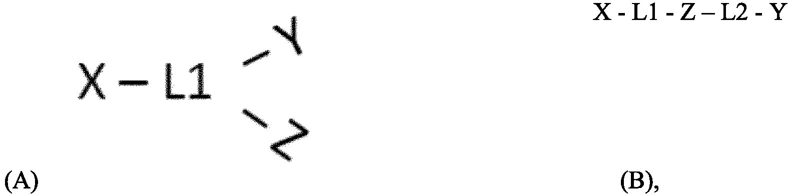 wobei
X eine reaktive Einheit ist, die insbesondere eine kovalente Bindung mit einem Analyten von Interesse bilden kann,
L1 und L2 unabhängig voneinander substituierte oder unsubstituierte Linker, insbesondere verzweigte oder lineare Linker sind,
Y eine Neutralverlusteinheit ist und
Z eine geladene Einheit ist, die mindestens eine permanent geladene Einheit umfasst,
insbesondere eine permanent geladene Einheit umfasst,
einschließlich eines Salzes davon, oder
wobei das Derivatisierungsreagens eine Verbindung der Formel PI umfasst: 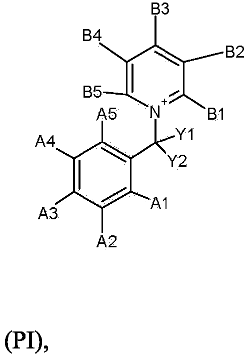
wobei einer der Substituenten B1, B2, B3, B4, B5 eine Kopplungsgruppe Q ist, die eine kovalente Bindung mit dem Analyten bilden kann,
wobei die anderen Substituenten A1, A2, A3, A4, A5, B1, B2, B3, B4, B5 jeweils unabhängig voneinander aus Wasserstoff, Halogen, Alkyl, N-Acylamino, N,N-Dialkylamino, Alkoxy, Thioalkoxy, Hydroxy, Cyano, Alkoxycarbonyl, Alkoxythiocarbonyl, Acyl, Nitro, Thioacyl, Aryloyl, Fluormethyl, Difluormethyl, Trifluormethyl, Trifluorethyl, Cyanomethyl, Cyanoethyl, Hydroxyethyl, Methoxyethyl, Nitroethyl, Acyloxy, Aryloyloxy, Cycloalkyl, Aryl, Heteroaryl, Heterocycloalkyl, Amino, einem Isotop oder Derivat davon ausgewählt sind,
wobei Y1 und Y2 jeweils unabhängig voneinander aus Wasserstoff, Methyl, Ethyl, Methoxy, substituierten aromatischen Verbindungen, unsubstituierten aromatischen Verbindungen, substituiertem Cycloalkyl, unsubstituiertem Cycloalkyl, substituierten heteroaromatischen Verbindungen, unsubstituierten heteroaromatischen Verbindungen, Amin ausgewählt sind oder wobei Y1 und Y2 eine Ringstruktur bilden, die aus substituiertem Cycloalkyl, unsubstituiertem Cycloalkyl, substituierten aromatischen Verbindungen, unsubstituierten aromatischen Verbindungen, substituierten heteroaromatischen Verbindungen, unsubstituierten heteroaromatischen Verbindungen ausgewählt sind, oder
wobei das Derivatisierungsreagens eine Verbindung der Formel DI umfasst: 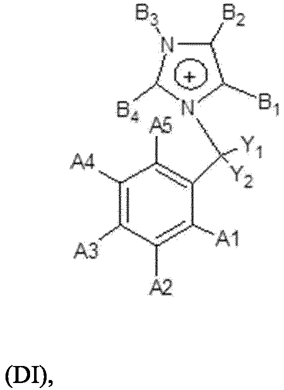
wobei einer der Substituenten B1, B2, B4 eine Kopplungsgruppe Q ist, die eine kovalente Bindung mit dem Analyten bilden kann,
wobei die anderen Substituenten A1, A2, A3, A4, A5, B1, B2, B4 jeweils unabhängig voneinander aus Wasserstoff, Halogen, Alkyl, N-Acylamino, N,N-Dialkylamino, Alkoxy, Thioalkoxy, Hydroxy, Cyano, Alkoxycarbonyl, Alkoxythiocarbonyl, Acyl, Nitro, Thioacyl, Aryloyl, Fluormethyl, Difluormethyl, Trifluormethyl, Trifluorethyl, Cyanomethyl, Cyanoethyl, Hydroxyethyl, Methoxyethyl, Nitroethyl, Acyloxy, Aryloyloxy, Cycloalkyl, Aryl, Heteroaryl, Heterocycloalkyl, Amino, einem Isotop oder Derivat davon ausgewählt sind,
wobei B3 aus Alkyl, Acetyl, Vinyl, substituierten aromatischen Verbindungen, unsubstituierten aromatischen Verbindungen, substituiertem Benzyl, unsubstituiertem Benzyl, substituiertem Cycloalkyl, unsubstituiertem Cycloalkyl, einem Isotop und Derivat davon ausgewählt ist,
wobei Y1 und Y2 jeweils unabhängig voneinander aus Wasserstoff, Methyl, Ethyl, Methoxy, substituierten aromatischen Verbindungen, unsubstituierten aromatischen Verbindungen, substituiertem Cycloalkyl, unsubstituiertem Cycloalkyl, substituierten heteroaromatischen Verbindungen, unsubstituierten heteroaromatischen Verbindungen, Amin ausgewählt sind oder wobei Y1 und Y2 eine Ringstruktur bilden, die aus substituiertem Cycloalkyl, unsubstituiertem Cycloalkyl, substituierten aromatischen Verbindungen, unsubstituierten aromatischen Verbindungen, substituierten heteroaromatischen Verbindungen, unsubstituierten heteroaromatischen Verbindungen ausgewählt sind, oder
wobei das Derivatisierungsreagens eine Verbindung der Formel CI umfasst: 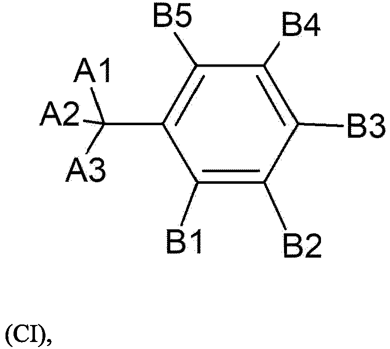
wobei einer der Substituenten B1, B2, B3, B4, B5 eine Kopplungsgruppe Q ist, die eine kovalente Bindung mit dem Analyten bilden kann,
wobei die anderen Substituenten A1, A2, B1, B2, B3, B4, B5 jeweils unabhängig voneinander aus Wasserstoff, Halogen, Alkyl, modifiziertem Alkyl, N-Acylamino, N,N-Dialkylamino, Alkoxy, Thioalkoxy, Hydroxy, Cyano, Alkoxycarbonyl, Alkoxythiocarbonyl, Acyl, Nitro, Thioacyl, Aryloyl, Fluormethyl, Difluormethyl, Trifluormethyl, Trifluorethyl, Cyanomethyl, Cyanoethyl, Hydroxyethyl, Methoxyethyl, Nitroethyl, Acyloxy, Aryloyloxy, Cycloalkyl, Aryl, Heteroaryl, Heterocycloalkyl, Amino, Schwefel, einem Isotop oder Derivat davon ausgewählt sind,
wobei A3 Ammonium, Pyridinium, Phosphonium oder Derivate davon umfasst, wobei, wenn A3 Ammonium ist und B1 oder B5 die Kopplungsgruppe Q ist, die Kopplungsgruppe Q ein C-Atom umfasst, das durch vier Einfach- oder Doppelbindungen von dem C-Atom des CA1A2A3-Substituenten getrennt ist, und die Kopplungsgruppe Q ein C-Atom umfasst, das durch fünf Einfach- oder Doppelbindungen von dem C-Atom des CA1A2A3-Substituenten getrennt ist.

9. Verfahren nach einem der vorstehenden Ansprüche, wobei der Analyt von Interesse aus der Gruppe ausgewählt ist, die aus Nukleinsäure, Aminosäure, Peptid, Protein, Metabolit, Hormonen, Fettsäure, Lipid, Kohlenhydrat, Steroid, Ketosteroid, Secosteroid, einem Molekül, das für eine bestimmte Modifikation eines anderen Moleküls charakteristisch ist, einer Substanz, die von dem Organismus internalisiert worden ist, einem Metaboliten von einer solchen Substanz und einer Kombination davon besteht, vorzugsweise wobei der Analyt von Interesse ein Peptid ist, das aus der Gruppe ausgewählt ist, die aus freien -NH₂- oder -COOH-Gruppen besteht.

10. Verwendung des Verfahrens nach einem der Ansprüche 1 bis 9 zum Bestimmen der Gegenwart oder des Gehalts eines Analyten von Interesse in einer Probe.

11. Verwendung eines Dotierstoffes in dem Verfahren nach einem der Ansprüche 1 bis 9, wobei der Dotierstoff zum Verstärken der Empfindlichkeit von massenspektrometrischem Nachweis eines Analyten von Interesse Formel I umfasst:
R1-SO₂-R2 (I),
wobei R1, R2 jeweils unabhängig voneinander aus Alkyl oder Aryl ausgewählt sind, wobei Alkyl ein bis sechs C-Atome umfasst, wobei Aryl ein bis sechs C-Atome umfasst.

12. Verwendung des Dotierstoffes nach Anspruch 11, wobei der Dotierstoff aus einem substituierten Sulfolan, einem unsubstituierten Sulfolan, einem substituierten Dibutylsulfon, einem unsubstituierten Dibutylsulfon, einem substituierten Diphenylsulfon oder einem unsubstituierten Diphenylsulfon ausgewählt ist.

13. Verwendung des Dotierstoffes nach einem der Ansprüche 11 bis 12, wobei der Dotierstoff frei von Sulfoxid, vorzugsweise frei von DMSO ist.

## Revendications

1. Procédé de détermination de la présence ou du taux d'un analyte d'intérêt dans un échantillon comprenant les étapes suivantes :
a) fourniture d'une première source d'ionisation par électronébulisation pour un premier flux d'écoulement analytique comprenant l'analyte d'intérêt,
b) fourniture d'une seconde source d'ionisation par électronébulisation pour un second flux d'écoulement analytique comprenant un dopant ou un réactif de dérivatisation, dans lequel chacun parmi le premier flux d'écoulement analytique et le second flux d'écoulement analytique est compris dans une forme gazeuse ou un aérosol,
c) intermélange du premier et du second flux d'écoulement analytique pour former un mélange ou un analyte d'intérêt dérivatisé, dans lequel le mélange comprend l'analyte d'intérêt et le dopant, et
d) détermination de la présence ou du taux d'un analyte d'intérêt dans l'échantillon en utilisant une spectrométrie de masse.

2. Procédé selon la revendication 1, dans lequel le procédé est réalisé pour améliorer le signal spectrométrique de masse de l'analyte d'intérêt.

3. Procédé selon la revendication 1 ou 2, dans lequel le premier flux d'écoulement analytique est intermélangé avec le second flux d'écoulement analytique après avoir quitté la première et la seconde source d'ionisation par électronébulisation.

4. Procédé selon l'une quelconque des revendications précédentes, dans lequel la première source d'ionisation par électronébulisation et la seconde source d'ionisation par électronébulisation sont agencées à au moins 10° à 180° selon une vue en plan sur les deux sources d'ionisation par électronébulisation et à au moins 10° à 180° selon une vue de face pour les sources d'ionisation par électronébulisation.

5. Procédé selon l'une quelconque des revendications précédentes, dans lequel le dopant est le DMSO ou NH₄F.

6. Procédé selon l'une quelconque des revendications précédentes, dans lequel le second flux d'écoulement comprend une concentration du dopant ou une concentration du réactif de dérivatisation dans la plage de 5 à 35 % (v/v).

7. Procédé selon l'une quelconque des revendications précédentes, dans lequel le réactif de dérivatisation est choisi dans le groupe constitué par le chlorure de dansyle, l'acide carbamique, l'ester de N-[2-[[[2-(diéthylamino)éthyl]amino]carbonyl]-6-quinoléinyl]-2,5-dioxo-1-pyrrolidinyle (RapiFluor-MS), les 1,2,4-triazoline-3,5-diones substituées en 4 (réactifs de type Cookson), un dérivé de la 4-phényl-1,2,4-triazolin-3,5-dione (Amplifex Diene), le 1-propanaminium, le composé 3-(aminooxy)-N,N,N-triméthyle comprenant un contre-ion approprié (Amplifex Keto), le chlorure d'acéthydrazide triméthylammonium (Girard T), l'hydrazide de chlorure de 1-(carboxyméthyl)pyridinium (Girard P) et la pyridiylamine.

8. Procédé selon l'une quelconque des revendications précédentes, dans lequel le réactif de dérivatisation comprend un composé de formule A ou B : 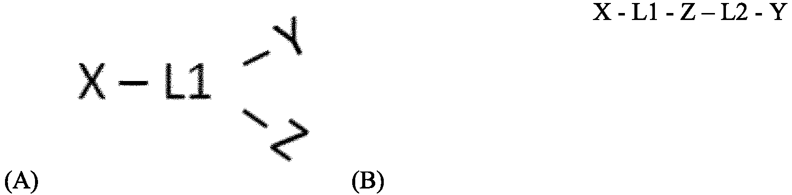 dans lequel
X représente un motif réactif, qui est en particulier capable de former une liaison covalente avec un analyte d'intérêt,
L1 et L2 sont indépendamment l'un de l'autre un lieur substitué ou non substitué, en particulier un lieur ramifié ou linéaire,
Y représente un motif de perte neutre, et
Z représente un motif chargé comprenant au moins une fraction chargée de manière permanente, en particulier comprenant une fraction chargée de manière permanente,
comprenant un sel quelconque de celui-ci, ou
dans lequel le réactif de dérivatisation comprend un composé de formule PI : 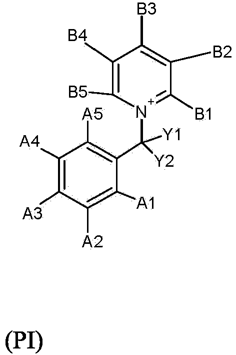
dans lequel l'un des substituants B1, B2, B3, B4, B5 représente un groupe de couplage Q, qui est capable de former une liaison covalente avec l'analyte,
dans lequel les autres substituants A1, A2, A3, A4, A5, B1, B2, B3, B4, B5 sont chacun indépendamment choisis parmi un hydrogène, un halogène, un alkyle, un N-acylamino, un N,N-dialkylamino, un alcoxy, un thioalcoxy, un hydroxy, un cyano, un alcoxycarbonyle, un alcoxythiocarbonyle, un acyle, un nitro, un thioacyle, un aryloyle, un fluorométhyle, un difluorométhyle, un trifluorométhyle, un trifluoroéthyle, un cyanométhyle, un cyanoéthyle, un hydroxyéthyle, un méthoxyéthyle, un nitroéthyle, un acyloxy, un aryloyloxy, un cycloalkyle, un aryle, un hétéroaryle, un hétérocycloalkyle, un amino, un isotope ou un dérivé de ceux-ci,
dans lequel Y1 et Y2 sont chacun indépendamment choisis parmi un hydrogène, un méthyle, un éthyle, un méthoxy, un aromatique substitué, un aromatique non substitué, un cycloalkyle substitué, un cycloalkyle non substitué, un hétéroaromatique substitué, un hétéroaromatique non substitué, une amine ou dans lequel Y1 et Y2 forment une structure cyclique, qui est choisie parmi un cycloalkyle substitué, un cycloalkyle non substitué, un aromatique substitué, un aromatique non substitué, un hétéroaromatique substitué, un hétéroaromatique non substitué, ou
dans lequel le réactif de dérivatisation comprend un composé de formule DI : 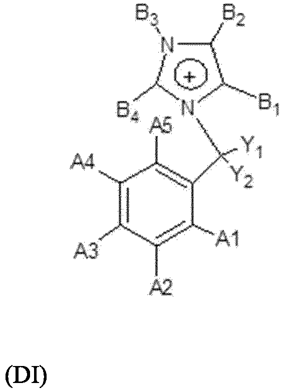
dans lequel l'un des substituants B1, B2, B4 représente un groupe de couplage Q, qui est capable de former une liaison covalente avec l'analyte,
dans lequel les autres substituants A1, A2, A3, A4, A5, B1, B2, B4 sont chacun indépendamment choisis parmi un hydrogène, un halogène, un alkyle, un N-acylamino, un N,N-dialkylamino, un alcoxy, un thioalcoxy, un hydroxy, un cyano, un alcoxycarbonyle, un alcoxythiocarbonyle, un acyle, un nitro, un thioacyle, un aryloyle, un fluorométhyle, un difluorométhyle, un trifluorométhyle, un trifluoroéthyle, un cyanométhyle, un cyanoéthyle, un hydroxyéthyle, un méthoxyéthyle, un nitroéthyle, un acyloxy, un aryloyloxy, un cycloalkyle, un aryle, un hétéroaryle, un hétérocycloalkyle, un amino, un isotope ou un dérivé de ceux-ci,
dans lequel B3 est choisi parmi un alkyle, un acétyle, un vinyle, un aromatique substitué, un aromatique non substitué, un benzyle substitué, un benzyle non substitué, un cycloalkyle substitué, un cycloalkyle non substitué, un isotope et un dérivé de ceux-ci,
dans lequel Y1 et Y2 sont chacun indépendamment choisis parmi un hydrogène, un méthyle, un éthyle, un méthoxy, un aromatique substitué, un aromatique non substitué, un cycloalkyle substitué, un cycloalkyle non substitué, un hétéroaromatique substitué, un hétéroaromatique non substitué, une amine ou dans lequel Y1 et Y2 forment une structure cyclique, qui est choisie parmi un cycloalkyle substitué, un cycloalkyle non substitué, un aromatique substitué, un aromatique non substitué, un hétéroaromatique substitué, un hétéroaromatique non substitué, ou
dans lequel le réactif de dérivatisation comprend un composé de formule CI : 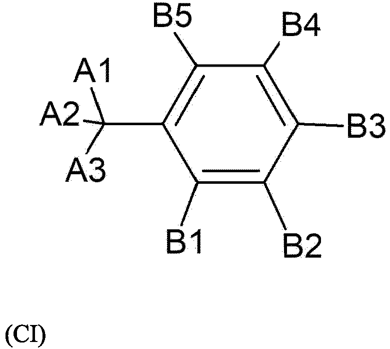
dans lequel l'un des substituants B1, B2, B3, B4, B5 représente un groupe de couplage Q, qui est capable de former une liaison covalente avec l'analyte,
dans lequel les autres substituants A1, A2, B1, B2, B3, B4, B5 sont chacun indépendamment choisis parmi un hydrogène, un halogène, un alkyle, un alkyle modifié, un N-acylamino, un N,N-dialkylamino, un alcoxy, un thioalcoxy, un hydroxy, un cyano, un alcoxycarbonyle, un alcoxythiocarbonyle, un acyle, un nitro, un thioacyle, un aryloyle, un fluorométhyle, un difluorométhyle, un trifluorométhyle, un trifluoroéthyle, un cyanométhyle, un cyanoéthyle, un hydroxyéthyle, un méthoxyéthyle, un nitroéthyle, un acyloxy, un aryloyloxy, un cycloalkyle, un aryle, un hétéroaryle, un hétérocycloalkyle, un amino, un soufre, un isotope ou un dérivé de ceux-ci,
dans lequel A3 comprend un ammonium, un pyridinium, un phosphonium ou des dérivés de ceux-ci,
dans lequel, dans le cas où A3 représente un ammonium et B1 ou B5 représente le groupe de couplage Q, le groupe de couplage Q comprend un atome de C, qui est séparé par quatre liaisons simples ou doubles de l'atome de C du substituant CA1A2A3 et le groupe de couplage Q comprend un atome de C, qui est séparé par cinq liaisons simples ou doubles de l'atome de C du substituant CA1A2A3.

9. Procédé selon l'une quelconque des revendications précédentes, dans lequel l'analyte d'intérêt est choisi dans le groupe constitué par un acide nucléique, un acide aminé, un peptide, une protéine, un métabolite, des hormones, un acide gras, un lipide, un glucide, un stéroïde, un cétostéroïde, un sécostéroïde, une molécule caractéristique d'une certaine modification d'une autre molécule, une substance qui a été internalisée par l'organisme, un métabolite d'une telle substance et une combinaison de ceux-ci, de préférence dans lequel l'analyte d'intérêt est un peptide choisi dans le groupe constitué par ceux présentant des groupes -NH₂ ou -COOH libres.

10. Utilisation du procédé selon l'une quelconque des revendications 1 à 9 pour la détermination de la présence ou du taux d'un analyte d'intérêt dans un échantillon.

11. Utilisation d'un dopant dans le procédé selon l'une quelconque des revendications 1 à 9, dans laquelle le dopant destiné à améliorer la sensibilité d'une détection spectrométrique de masse d'un analyte d'intérêt comprend la formule I :
R1-SO₂-R2 (I)
dans laquelle R1, R2 sont chacun indépendamment choisis parmi un alkyle ou un aryle, dans laquelle l'alkyle comprend un à six atomes de C, dans laquelle l'aryle comprend un à six atomes de C.

12. Utilisation du dopant selon la revendication 11, dans laquelle le dopant est choisi parmi un sulfolane substitué, un sulfolane non substitué, une dibutylsulfone substituée, une dibutylsulfone non substituée, une diphénylsulfone substituée ou une diphénylsulfone non substituée.

13. Utilisation du dopant selon l'une quelconque des revendications 11 à 12, dans laquelle le dopant est exempt de sulfoxyde, de préférence exempt de DMSO.
